# EUROPEAN PATENT SPECIFICATION

(11) **EP 2 970 436 B1**
(45) Date of publication and mention of the grant of the patent: **05.09.2018**
(21) Application number: 14720003.4
(22) Date of filing: 14.03.2014
(51) Int. Cl.: C07K 16/00, A61K 39/00, C07K 16/28

(54) **FC VARIANTS**
FC-VARIANTEN
VARIANTS DE FC

(30) Priority: 15.03.2013 US 201361791624 P
(43) Date of publication of application: 20.01.2016
(73) Proprietor: AbbVie Biotherapeutics Inc., Redwood City, CA 94063 (US)
(72) Inventor: HARDING, Fiona A., Mountain View, California 94041 (US); HINTON, Paul R., Sunnyvale, California 94085 (US); XIONG, Mengli, Union City, California 94587 (US); RAZO, Olivia Jennifer, Newark, California 94560 (US); YE, Shiming, Palo Alto, California 94306 (US)
(74) Representative: J A Kemp
(86) International application number: PCT/US2014/029585
(87) International publication number: WO 2014/144960

(56) References cited:
- EP-A1- 2 679 681
- WO-A1-2011/120134
- WO-A2-2004/099249
- WO-A2-2004/100898
- WO-A2-2006/034488
- WO-A2-2007/024249
- WO-A2-2013/109279
- US-A1- 2007 231 329
- US-A1- 2008 112 961
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02) , pages 6591-6604, XP002271092, ISSN: 0021-9258, DOI: 10.1074/JBC.M009483200
- PRESTA L G ET AL: "Engineering therapeutic antibodies for improved function", BIOCHEMICAL SOCIETY TRANSACTIONS, PORTLAND PRESS LTD, GB, vol. 30, no. 4, 1 August 2002 (2002-08-01) , pages 487-490, XP002432766, ISSN: 0300-5127, DOI: 10.1042/BST0300487

## Description

### 3. BACKGROUND

The fragment crystallizable ("Fc") region of an antibody is composed of two identical protein fragments, derived from the second and third constant domains of the antibody's two heavy chains. Fc regions bind to receptors on immune cells known as Fc receptors ("FcRs"), leading to both activating and inhibitory signals. For example, the FcγRIIIA (also known as CD16 or CD16a) is found on natural killer cells and macrophages, and has a low affinity for Fc regions. Binding of Fc ligand to an FcγRIIIA receptor can result in induction of antibody-dependent cell-mediated cytotoxicity (ADCC) and induction of cytokine release by macrophages. In contrast, the FcγRIIB receptor (also known as CD32b) is found on macrophages, neutrophils, B cells and eosinophils, and binding of Fc ligand to an FcγRIIB receptor inhibits cell activity.

The differences in downstream signaling affected by different FcRs are based on structural differences. Within the Fc gamma receptor ("FcγR") family-including FcγRI (CD64), FcγRIIA (CD32), FcγRIIIA (CD16a), and FcγRIIIB (CD16b)- Fc receptors generate activation signals via a motif known as an Immunoreceptor Tyrosine-based Activation Motif (ITAM). FcγRIIA, FcγRI, and FcγRIIIA all produce activating signals through their ITAMs, or by interaction with an ITAM-containing subunit. Alternatively, an Fc receptor can contain an Immunoreceptor Tyrosine-based Inhibitory Motif (ITIM) that generates inhibitory signals. FcγRIIB1 and FcγRIIB2, alternatively spliced forms of FcγRIIB (collectively referred to as "FcγRIIB") have ITIM sequences, and thus function as inhibitory Fc receptors (*see* Blank et al., 2009, Immunol Rev. 232(1):59-71).

By altering the Fc regions of antibodies, improvements can be made to increase antibody therapeutic efficacy, increase antibody half-life, and reduce unwanted side effects.

### 4. SUMMARY

Because FcγRIIIA generates signals that activate immune cells, in particular cells that mediate ADCC, it is believed that decreasing Fc binding to FcγRIIIA will result in decreased immune cell activation and reduced levels of ADCC. Moreover, because FcγRIIB generates signals that inhibit immune cells, the dual effect of increasing Fc binding to FcγRIIB coupled with decreasing Fc binding to FcγRIII will result in greater inhibition of immune cell activation as compared to modulating binding of a single receptor. The present inventors have identified single amino acid substitutions or point mutations in the CH2 domains of Fc molecules that impact binding to both Fc receptors. For example, as will be discussed in more detail below, amino acid substitutions at a single position (*e.g.,* V263) can significantly increase binding to FcγRIIB and decrease binding to FcγRIIIA. The incorporation of such amino acid substitutions into antibodies and other Fc-based therapeutic molecules can result in variant polypeptides with a greater inhibition of immune cell activation as compared to substitutions that modulate binding to a single receptor. The variant polypeptides are particularly suited for treatment of indications for which induction of immune activation is not desirable, for example in the treatment of immune disorders.

In one embodiment of the present invention, there is provided an antibody comprising a CH2 domain of SEQ ID NO:2, which has relative to the CH2 domain of SEQ ID NO:2 (a) a substitution or set of substitutions according to the EU index as in Kabat selected from: (i) V273E, (ii) V273F, (iii) V273M, (iv) V273S, (v) V273Y, (vi) V263L and V273E, (vii) V263L and V273F, (viii) V263L and V273M, (ix) V263L and V273S, and (x) V263L and V273Y; (b) the substitution V263L according to the EU index as in Kabat, wherein the antibody binds specifically to CD40; or (c) the substitution V263L according to the EU index as in Kabat, wherein the antibody binds specifically to a costimulatory molecule selected from CD28, PD-1, CTLA-4, CD80, CD86, TIM3, OX40, 4-1BB, GITR, CD27, B7-H4, and DC-SIGN; optionally wherein the CH2 domain is part of an Fc domain comprising T250Q and M428L according to the EU index as in Kabat.

In another embodiment of the present invention, there is provided a conjugate compound comprising the antibody of the invention linked to an effector moiety or a detectable label.

In a further embodiment of the present invention, there is provided a pharmaceutical composition comprising the antibody of the invention and a pharmaceutically acceptable carrier, or the conjugate compound of the invention.

In another embodiment of the present invention, there is provided a vector comprising a nucleic acid comprising a nucleotide sequence encoding the antibody of the invention.

In a further embodiment of the present invention, there is provided a prokaryotic host cell comprising the vector of the invention.

In another embodiment of the present invention, there is provided a method of producing an antibody, comprising: (a) culturing a eukaryotic host cell engineered to express a nucleic acid comprising a nucleotide sequence encoding the antibody of the invention and (b) recovering the antibody.

In a further embodiment of the present invention, there is provided an antibody of the invention, a conjugate compound of the invention, or a pharmaceutical composition of the invention, for use in treating an immune disorder or a cancer in a patient in need thereof.

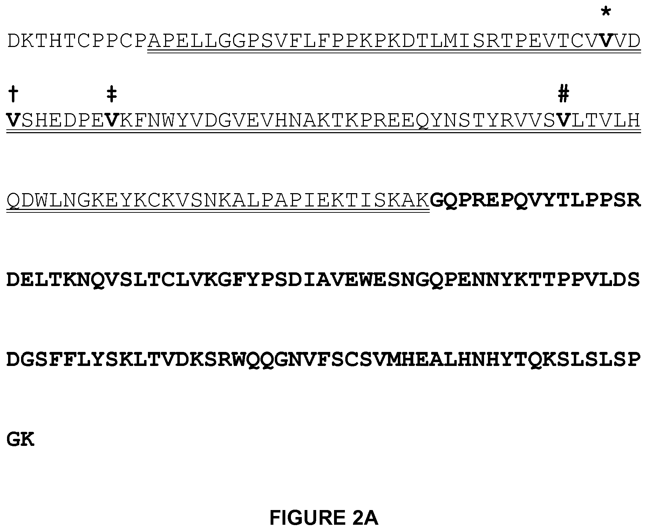

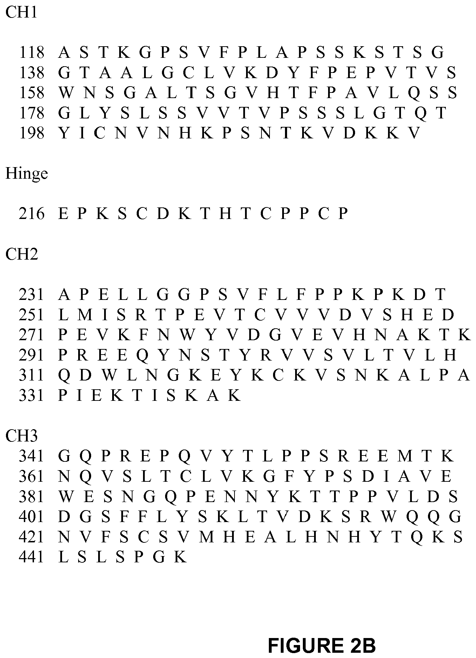

Accordingly, in one aspect, the present disclosure provides polypeptides comprising modified (or variant) CH2 domains or entire Fc domains (collectively referred to as "variant polypeptides" or "variant Fc polypeptides") that include amino acid substitutions that increase binding to FcγRIIB and/or reduced binding to FcγRIIIA as compared to the binding of a corresponding wild-type CH2 (SEQ ID NO:2) or Fc region (SEQ ID NO:1). A polypeptide of the disclosure can be a monomer or multimer (*e.g.,* dimer or tetramer), each monomeric unit comprising one or more CH2 or Fc domains. A polypeptide of the disclosure is typically an antibody or an Fc fusion protein comprising a variant CH2 or Fc domain of the disclosure. A variant CH2 or variant Fc domain of the present disclosure typically includes one or more substitutions at position 263, position 266, position 273, and position 305, wherein the numbering of the residues in the Fc domain is that of the EU index as in Kabat. These amino acid positions are indicated by asterisk (*), dagger (†), double dagger (‡), and the number sign (#), respectively, in Figure 2.

Thus, in one aspect, the present disclosure provides polypeptides comprising a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:2. Relative to the CH2 domain of SEQ ID NO:2, disclosed polypeptides can comprise one or more substitutions selected from: (a) a V263 substitution that increases affinity towards FcγRIIB and decreases affinity towards FcγRIIIA; (b) a V266 substitution that increases affinity towards FcγRIIB and decreases affinity towards FcγRIIIA; and (c) a V273 substitution that increases affinity towards FcγRIIB and decreases affinity towards FcγRIIIA.

We also disclose polypeptides comprising one or more substitutions selected from V263L, V266L, V273C, V273E, V273F, V273L, V273M, V273S, V273Y, V305K, and V305W, relative to the CH2 domain of SEQ ID NO:2. In specific aspects of the disclosure, the one or more substitutions of the CH2 domain may be selected from V263L, V273E, V273F, V273M, V273S, and V273Y.

In another aspect, the present disclosure provides polypeptides comprising a variant CH2 domain which has up to 6, up to 5, up to 4, up to 3, up to 2 substitutions, or a single amino acid substitution as compared to an CH2 domain of SEQ ID NO:2, including one or more substitutions selected from: (a) a V263 substitution that increases affinity towards FcγRIIB and decreases affinity towards FcγRIIIA; (b) a V266 substitution that increases affinity towards FcγRIIB and decreases affinity towards FcγRIIIA; and (c) a V273 substitution that increases affinity towards FcγRIIB and decreases affinity towards FcγRIIIA.

Polypeptides of the disclosure can also comprise a variant CH2 domain which has up to 6, up to 5, up to 4, up to 3, up to 2 substitutions, or a single amino acid substitution, as compared to an CH2 domain of SEQ ID NO:2, including one or more substitutions selected from V263L, V266L, V273C, V273E, V273F, V273L, V273M, V273S, V273Y, V305K, and V305W. In specific aspects of the disclosure the one or more substitutions of the CH2 domain may be selected from V263L, V273E, V273F, V273M, V273S, and V273Y.

As discussed in detail herein, the CH2 domain is a component of the Fc domain of an antibody. Accordingly, in one aspect of the disclosure polypeptides are provided that comprise an Fc domain, said Fc domain comprising a CH2 domain of the disclosure. In some aspects of the disclosure, the Fc domain may have up to 20, up to 15, up to 12, up to 10, up to 9, up to 8, up to 7, up to 6, up to 5 or up to 4 amino acid substitutions as compared to the CH2 domain of the Fc domain of SEQ ID NO:1. Overall, the Fc domain of the polypeptide can have at least 70%, at least 75%, at least 80%, at least 85%, at least 90%, at least 95%, at least 96%, at least 97%, at least 98% or at least 99% sequence identity to the Fc domain of SEQ ID NO:1.

Skilled artisans will appreciate that disclosed Fc domains can comprise any of the one or more CH2 substitutions described herein. Thus, polypeptides are provided including those in which comprise a V263 substitution (*e.g.,* V263L), a V266 substitution (*e.g.,* V266L), a V273 substitution (*e.g.,* V273E, V273F, V273L, V273M, V273S, or V273Y), or a V305 substitution (*e.g.,* V305K or V305W).

We also disclose polypeptides comprising a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:2, and which has relative to the CH2 domain of SEQ ID NO:2 one or more substitutions selected from: a V263 substitution that increases affinity towards FcγRIIB and decreases affinity towards FcγRIIIA; and a V273 substitution that increases affinity towards FcγRIIB and decreases affinity towards FcγRIIIA.

We further disclose polypeptides comprising a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:2, and which has relative to the CH2 domain of SEQ ID NO:2: the substitution V263L; and/or a V273 substitution selected from V273C, V273E, V273F, V273L, V273M, V273S, V273Y.

We also disclose polypeptides comprising a variant CH2 domain which has up to 6, up to 5, up to 4, up to 3, up to 2 substitutions, or a single amino acid substitution, as compared to an CH2 domain of SEQ ID NO:2, including one or more substitutions selected from: a V263 substitution that increases affinity towards FcγRIIB and decreases affinity towards FcγRIIIA; a V266 substitution that increases affinity towards FcγRIIB and decreases affinity towards FcγRIIIA; and a V273 substitution that increases affinity towards FcγRIIB and decreases affinity towards FcγRIIIA.

We further disclose polypeptides comprising a variant CH2 domain which has up to 6, up to 5, up to 4, up to 3, up to 2 substitutions, or a single amino acid substitution, as compared to an CH2 domain of SEQ ID NO:2, including one or more substitutions selected from: the substitution V263L; and/or a V273 substitution selected from V273C, V273E, V273F, V273L, V273M, V273S, V273Y.

Fc domains are known to mediate Fc effector functions, as described in Section 3.5. Accordingly, the disclosure provides polypeptides that further comprise one or more additional substitutions or combinations of substitutions that modify Fc effector function. Typically, Fc effector functions that can be modified include (a) reduction or increase in binding to FcRN; (b) reduction or increase in binding to FcγRI; (c) reduction or increase in binding to FcγRIIA or FcγRIIB; (d) reduction or increase inbinding to FcγRIIIA; or (e) a combination of two, three, four or all of the foregoing.

An exemplary substitution known to modify Fc effector function is the Fc substitution M428L, which can occur in combination with the Fc substitution T250Q. Additionally, an Fc domain of the disclosure can comprise one or more additional substitutions selected from Table 1, as compared to the Fc domain of SEQ ID NO:1.

In one aspect, the disclosure provides polypeptides that are antibodies, discussed in further detail in Section 3.1. These antibodies can be human or humanized antibodies. In typical embodiments of the disclosure, an antibody specifically binds to CD40, CD25, CD3, an HLA molecule, a costimulatory molecule, a cytokine (*e.g.,* TNF-α or IL-2), a chemokine, an adhesion molecule (*e.g.,* α4 integrin), an activation markers, or an immunomodulatory protein. The costimulatory molecule can be CD28, PD-1, CTLA-4, CD80, CD86, TIM3, OX40, 4-1BB, GITR, CD27, B7-H4, or DC-SIGN. In some aspects of the disclosure the immunomodulatory protein may be a cell surface molecule. Alternatively, in other aspects of the disclsure the immunomodulatory protein may be a soluble molecule.

In certain aspects of the disclosure, the antibody can specifically bind to CD25. Alternatively, in other aspects of the disclosure the antibody can specifically bind to CD40.

Polypeptides of the disclosure also include Fc fusion proteins in which the CH2 domain is part of an Fc domain operably linked to at least one fusion partner. Fc fusion proteins are discussed in detail in Section 3.3. In such Fc proteins, said at least one fusion partner can be the extracellular domain ("ECD") of TNF receptor II; the first ECD of lymphocyte function-associated antigen 3 (LFA-3); the ECD of human cytotoxic T lymphocyte associated molecule-4 (CTLA-4); the C-terminus of the IL-1R accessory protein ligand binding region; the N-terminus of the IL-1RI ECD; peptide thrombopoietin (TPO) mimetic; ECD of CTLA-4 with the two amino acid substitutions L104E and A29Y; and the ECDsof VEGF receptor 1 and/or the ECD of VEGF receptor 2.

In another aspect, the disclosure provides conjugate compounds comprising polypeptides the disclosure linked to an effector moiety or a detectable label. Conjugate compounds are discussed further in section 3.6. In some aspects of the disclosure the conjugate compound may comprise a polypeptide linked to a detectable label, such as a radioactive compound, a fluorescent compound, an enzyme, a substrate, an epitope tag or a toxin. In some aspects of the disclosure the conjugate compound may comprise a polypeptide linked to an effector moiety, such as a cytotoxic agent. Skilled artisans will appreciate the various cytotoxic agents that can be linked to polypeptides of the disclosure, including an auristatin, a DNA minor groove binding agent, a DNA minor groove alkylating agent, an enediyne, a duocarmycin, a maytansinoid or a vinca alkaloid. Other exemplary cytotoxic agents are anti-tubulins, AFP, MMAF, or MMAE.

The present disclosure further provides pharmaceutical compositions comprising polypeptides of the disclosure and a pharmaceutically acceptable carrier or a conjugate compound of the disclosure. Pharmaceutical compositions and methods of treatment are discussed in detail in Section 3.7.

Nucleic acids comprising nucleotide sequences encoding the polypeptides of the disclosure are provided herein, as are vectors comprising nucleic acids. Additionally, prokaryotic and eukaryotic host cells transformed with a vector comprising a nucleotide sequence encoding a disclosed polypeptide are provided herein, as well as eukaryotic (such as mammalian) host cells engineered to express the nucleotide sequences. Methods of producing polypeptides, by culturing host cells and recovering the polypeptides are also provided, and discussed further in section 3.4, below.

Skilled artisans will appreciate that the polypeptides of the disclosure are useful in the treatment of various diseases or disorders such as an immune disorder or cancer for which it would be suitable to administer to a patient in need thereof an appropriate polypeptide, pharmaceutical composition, or conjugate compound of the disclosure.

In some aspects of the disclosure the polypeptide can be an anti-CD40 antibody useful for treatment of a cancer. In some aspects of the disclosure the cancer may be a hematological cancer optionally selected from chronic lymphocytic leukemia, Burkitt's lymphoma, multiple myeloma, a T cell lymphoma, Non-Hodgkin's Lymphoma, Hodgkin's Disease, Waldenstrom's macroglobulinemia or Kaposi's sarcoma. In particular embodiments, the cancer is a solid tumor, optionally selected from ovarian cancer, breast cancer, lung cancer, melanoma, pancreatic cancer, and renal cancer. Exemplary VL and VH sequences of an anti-CD40 antibody are provided as SEQ ID NO:3 and SEQ ID NO:4, respectively. In specific aspects of the disclosure the anti-CD40 antibody may be a multi-specific antibody.

We also disclose a polypeptide that is an anti-CD20 antibody useful for treatment of an immune disorder which is rheumatoid arthritis or multiple sclerosis. Exemplary VL and VH sequences of an anti-CD20 antibody are provided as SEQ ID NO:5 and SEQ ID NO:6, respectively.

We also disclose a polypeptide that is an anti-CD25 antibody useful for treatment of an immune disorder which is multiple sclerosis, asthma, psoriasis, uveitis, ocular inflammation or organ transplant rejection or of a cancer which is human T cell leukemia virus-1 associated T-cell leukemia. Exemplary VL sequences of an anti-CD25 antibody include SEQ ID NO:7 and SEQ ID NO:9. Exemplary VL sequences of an anti-CD25 antibody include SEQ ID NO:8 and SEQ ID NO:10.

We further disclose a polypeptide that is an anti-TNFa antibody useful for treatment of an immune disorder which is rheumatoid arthritis, juvenile idiopathic arthritis, psoriatic arthritis, ankylosing spondylitis, Crohn's disease, ulcerative colitis or plaque psoriasis. Exemplary VL sequences of an anti-TNFα antibody include SEQ ID NO:11 and SEQ ID NO:13. Exemplary VL sequences of an anti-TNFα antibody include SEQ ID NO:12 and SEQ ID NO:14.

We also disclose a polypeptide that is an anti-IL-6 receptor antibody useful for treatment of an immune disorder which is rheumatoid arthritis or Castleman's Disease. Exemplary VL and VH sequences of an anti-IL-6 receptor antibody are provided as SEQ ID NO:15 and SEQ ID NO:16, respectively. The polypeptide can also be an anti-α4-integrin antibody useful for treatment of an immune disorder which is multiple sclerosis. In some aspects of the disclosure the polypeptide can be an anti-IL-1 antibody useful for treatment of an immune disorder which is Cryopyrin-Associated Periodic Syndromes ("CAPS"). Exemplary VL and VH sequences of an anti-IL-6 receptor antibody are provided as SEQ ID NO:17 and SEQ ID NO:18, respectively. The polypeptide can also be an anti-BAFF antibody and useful for treatment of an immune disorder which is systemic lupus erythmatosis or allergy. Exemplary VL and VH sequences of an anti-BAFF antibody are provided as SEQ ID NO:19 and SEQ ID NO:20, respectively.

A more complete appreciation of the various inventions disclosed herein, and many of the attendant advantages thereof, is provided by the detailed description that follows.

As used herein throughout the specification and in the appended claims, the following terms and expressions are intended to have the following meanings:

The indefinite articles "a" and "an" and the definite article "the" are intended to include both the singular and the plural, unless the context in which they are used clearly indicates otherwise.

"At least one" and "one or more" are used interchangeably to mean that the article may include one or more than one of the listed elements.

Unless otherwise indicated, it is to be understood that all numbers expressing quantities, ratios, and numerical properties of ingredients, reaction conditions, and so forth, used in the specification and claims are contemplated to be able to be modified in all instances by the term "about."

### 5. BRIEF DESCRIPTION OF THE FIGURES

**FIGURE 1** provides a schematic representation of a native IgG. Disulfide bonds are represented by heavy lines between CH1 and CL domains and the two CH2 domains. V is variable domain; C is constant domain; L stands for light chain and H stands for heavy chain.
**FIGURES 2A-2B****.** **FIGURE 2A** provides the sequence of a wild type Fc domain, from human IgG1 (SEQ ID NO:1). Within the Fc domain the CH2 domain (whose sequence is APELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAK; SEQ ID NO:2) is double underlined and the CH3 domain (whose sequence is GQPREPQVYTLPPSRDELTKNQVSLTCLVKGFYPSDIAVEWESNGQPENNYKTTPPVLDSDG SFFLYSKLTVDKSRWQQGNVFSCSVMHEALHNHYTQKSLSLSPGK; SEQ ID NO:21) is bolded. Residues 263, 266, 273, and 305 are indicated by asterisk (*), dagger (†), double dagger (‡), and the number sign (#), respectively. **FIGURE 2B** shows the amino acid sequences and the numbering of the amino acids in the CHI, hinge, CH2 and CH3 domains. The full-length sequence of the CHI, hinge, CH2 and CH3 domains in **FIGURE 2B** is referred to as SEQ ID NO: 38.
**FIGURE 3** provides a schematic of complexes used to measure binding of polypeptides of the disclosure to Fc receptors.
**FIGURE 4** provides FACS titration curves and EC₅₀ measurements for binding of wild-type hu1D10 to (A) FcγRIIB1 and (B) FcγRIIIA.
**FIGURE 5** provides a representative FACS sort result.
**FIGURE 6** provides a plot displaying enrichment ratios for individual clones significantly increased for FcγRIIB binding and decreased for binding to FcγRIIIA.
**FIGURE 7** provides positions and substitutions that were identified as having significantly increased FcγRIIB binding and decreased binding to FcγRIIIA. The enrichment ratios for each mutant are tabulated.
**FIGURE 8** provides single-point FACS data for binding of polypeptides of the disclosure to FcγRIIB.
**FIGURE 9** provides single-point FACS data for binding of polypeptides of the disclosure to FcγRIIIA.
**FIGURES 10A-10B** provides confirmatory data showing that polypeptides of the disclosure demonstrated higher maximal binding to FcγRIIB than the wild-type antibody. **FIGURE 10A** shows binding curves of WT and variant Fc regions to FcγRIIB; **FIGURE 10B** shows the EC50 of binding for each variant and the fold over wild type binding.
**FIGURES 11A-11B** provides confirmatory data showing that polypeptides of the disclosure demonstrated higher maximal binding to FcγRIIIA than the wild-type antibody. **FIGURE 11A** shows binding curves of WT and variant Fc regions to FcγRIIIA; **FIGURE 11B** shows the EC50 of binding for each variant and the fold over wild type binding.
**FIGURE 12** provides FACS data from testing polypeptides of the disclosure using a non-radioactive ADCC assay.
**FIGURES 13A-13D****.** **FIGURE 13A** provides data showing percent cytotoxicity graphed against IgG concentration to determine the EC50. **FIGURE 13B** shows FcγRIIB up-mutants having some ADCC activity, though lower than wild-type. **FIGURE 13C** shows polypeptides with little to no ADCC activity. **FIGURE 13D** compares the non-ADCC hu1D10 polypeptides to previously known substitutions that result in decreased binding to FcγRIIIA (S267E, L328F, double mutant "SELF").
**FIGURES 14A-14B. FIGURE 14A** provides results for induction of ADCC for polypeptides of the disclosure using a chromium release assay. **FIGURE 14B** provides symbol key for FIGURE 14A.
**FIGURES 15A-15C** provides results for dendritic cell activation for polypeptides of the disclosure using monocyte-derived immature dendritic cells. **FIGURE 15A** shows dendritic cell activation by ADCC-inducing variants. **FIGURE 15B** shows dendritic cell activation by non-ADCC-inducing variants. **FIGURE 15C** shows the EC50 for IL-12 induction.
**FIGURE 16** shows Fc variants with lowest ADCC activity with retained/improved FcγRIIB binding in bold font.

### 6. DETAILED DESCRIPTION

### 6.1. Fc Variant Polypeptides

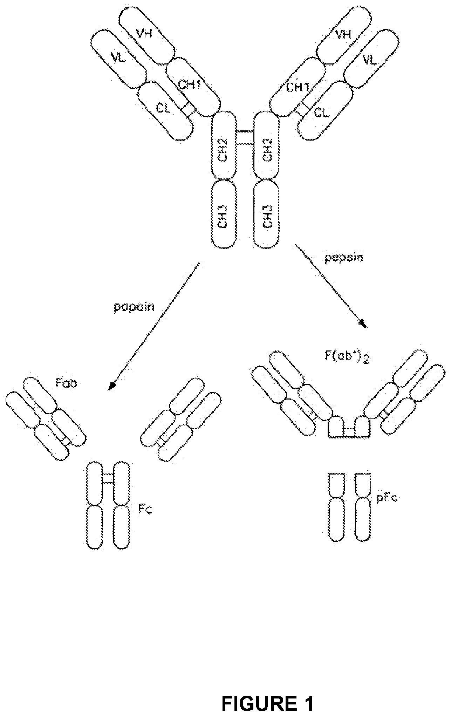

Fc domains of immunoglobulin are involved in non-antigen binding function and have several effector functions mediated by binding of effector molecules. As illustrated in Figure 1, Fc domains are composed of two main domains, the CH2 domain and the CH3 domain, and have a small hinge region N-terminal to the CH2 domain. The present disclosure provides polypeptides comprising modified CH2 domains (and modified Fc domains comprising modified CH2 domains), collectively referred to herein as variant polypeptides, Fc variants, or simply variants or polypeptides. The variant polypeptides are typically antibodies or antibody fragments (referred to herein collectively as antibody variants) or Fc fusion proteins.

As used herein, numbering of antibody amino acid residues is done according to Kabat EU nomenclature unless otherwise indicated.

As used herein, the term "Fc domain" refers to a C-terminal region of an immunoglobulin heavy chain. Although the generally accepted boundaries of the Fc domain of an immunoglobulin heavy chain might vary, the human IgG heavy chain Fc domain is usually defined to stretch from an amino acid residue at position Cys226, or from Pro230, to the carboxyl-terminus thereof. In some aspects of the disclosure variants may comprise only portions of the Fc domain and can include or not include the carboxyl-terminus. The Fc domain of an immunoglobulin generally comprises two constant domains, CH2 and CH3. The Fc variant polypeptides of the disclosure typically include at a CH2 domain and oftentimes also include a CH3 domain.

As used herein, the "CH2 domain" (also referred to as "Cγ2" domain) generally comprises the stretch of residues that extends from about amino acid 231 to about amino acid 340 in an Fc domain (*e.g.,* in the human IgG Fc domain). The CH2 domain is unique in that it is not closely paired with another domain. Rather, two N-linked branched carbohydrate chains are interposed between the two CH2 domains of an intact native IgG molecule.

As used herein, the "CH3 domain" (also referred to as "Cγ3" domain) generally comprises the stretch of residues C-terminal to a CH2 domain in an Fc domain (*e.g.,* from about amino acid residue 341 to about amino acid residue 447 of a human IgG Fe region).

The terms "Fc receptor" and "FcR" are used to describe a receptor that binds to an Fc domain (*e.g.* the Fc domain of an antibody or antibody fragment). Portions of Fc receptors are specifically contemplated in some embodiments of the present invention. In preferred aspects of the disclosure the FcR can be a native sequence human FcR. In other preferred aspects of the disclosure the FcR can also be one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine based activation motif (ITAM) in its cytoplasmic domain. Inhibiting receptor FcγRIIB contains an immunoreceptor tyrosine-based inhibition motif (ITIM) in its cytoplasmic domain. Other FcRs, including those to be identified in the future, are encompassed by the term "FcR" herein. The term also includes the neonatal receptor, FcRn.

The polypeptides of the disclosure comprise an Fc variant domain having an amino acid sequence substantially homologous to all or part of a human immunoglobulin constant region, preferably an IgG C-domain.

Numerous sequences for human C regions have been published; see, *e.g.,* Clark, 1997, Chem. Immunol. 65:88-110. Other sequences for human immunoglobulin heavy chains can be obtained from the SwissProt and PIR databases using Lasergene software (DNAStar Limited, London UK) under accession numbers A93433, B90563, A90564, B91668, A91723 and A02146 for human Igγ-1 chain C region, A93906, A92809, A90752, A93132, A02148 for human Ig γ-2 chain C region, A90933, A90249, A02150 for human Igγ-4 chain C region, and A23511 for human Igγ-3 chain C region. An exemplary Fc domain has the amino acid sequence of SEQ ID NO:1.

In various aspects of the disclosure the amino acid sequence of the Fc variant domain can share at least about 70%, 75%, 80%, 85%, 90%, 95%, 96%, 97%, 98% or 99% sequence identity with the reference any of the foregoing Fc domains. In preferred aspects of the disclosure the reference Fc domain can comprise SEQ ID NO:1.

Sequence comparisons are typically performed by comparing sequences over a "comparison window" to identify and compare local regions of sequence similarity. A "comparison window" refers to a conceptual segment of typically 12 contiguous residues that is compared to a reference sequence. The comparison window may comprise additions or deletions (*i.e.,* gaps) of about 20% or less as compared to the reference sequence (which does not comprise additions or deletions) for optimal alignment of the respective sequences. Optimal alignment of sequences for aligning a comparison window may be conducted by computerised implementations of algorithms (Geneworks program by Intelligenetics; GAP, BESTFIT, FASTA, and TFASTA in the Wisconsin Genetics Software Package Release 7.0, Genetics Computer Group, 575 Science Drive Madison, Wis., USA) or by inspection and the best alignment (*i.e.,* resulting in the highest percentage homology over the comparison window) generated by any of the various methods selected. Reference also may be made to the BLAST family of programs as for example disclosed by Altschul et al., 1997, Nucl. Acids Res. 25(17):3389-402.

The present disclosure provides polypeptides comprising a modified Fc domain wherein the binding of the polypeptide to a first Fc receptor, *e.g.,* FcγRIIB, is increased compared to that of the wild-type Fc domain, and the binding of the polypeptide to a second Fc receptor, *e.g.,* FcγRIIIA, is decreased compared to that of an antibody having a wild-type Fc domain. The polypeptide can be an antibody or an Fc fusion protein.

The Fc variant polypeptides can comprise a single substitution that results in both increased binding to FcγRIIB and decreased binding to FcγRIIIA, as compared to that of a polypeptide having a wild-type Fc domain.

The Fc variant polypeptides can comprise a variant constant region heavy chain domain 2 ("CH2") having at least one substitution selected from V263L, V266L, V273C, V273E, V273F, V273L, V273M, V273S, V273Y, V305K, and V305W as compared to a CH2 domain of SEQ ID NO:2. The variant CH2 domain preferably has at least 70%, at least 75%, at least 80%, at least 85%, at least 90%, at least 95%, at least 96%, at least 97%, at least 98%, or at least 99% sequence identity to the CH2 domain of SEQ ID NO:2. In various aspects of the disclosure the CH2 domain can include at least one substitution selected from V263L, V273E, V273F, V273M, V273S, and V273Y.

In various aspects of the disclosure the variant CH2 domain may have altogether up to 20, up to 15, up to 12, up to 10, up to 9, up to 8, up to 7, up to 6, up to 5 or up to 4 amino acid substitutions as compared to a CH2 domain of SEQ ID NO:2. In some aspects of the disclosure the CH2 domain can have no more than 6, no more than 5, no more than 4, no more than 3, or no more than 2 amino acid substitutions as compared to the CH2 domain of SEQ ID NO:2.

The Fc variant polypeptides can comprise a variant Fc region comprising a CH2 domain having at least one substitution selected from V263L, V266L, V273C, V273E, V273F, V273L, V273M, V273S, V273Y, V305K, and V305W as compared to a CH2 domain of SEQ ID NO:2. The variant Fc region preferably has at least 70%, at least 75%, at least 80%, at least 85%, at least 90%, at least 95%, at least 96%, at least 97%, at least 98%, or at least 99% sequence identity to the Fc region of SEQ ID NO:1. In various aspects of the disclosure the CH2 domain may include at least one substitution selected from V263L, V273E, V273F, V273M, V273S, and V273Y.

In some aspects of the disclosure the variant Fc region domain may have altogether up to 20, up to 15, up to 12, up to 10, up to 9, up to 8, up to 7, up to 6, up to 5 or up to 4 amino acid substitutions as compared to an Fc domain of SEQ ID NO:1. In some aspects of the disclosure the variant Fc region can have no more than 6, no more than 5, no more than 4, no more than 3, or no more than 2 amino acid substitutions as compared to the Fc region of SEQ ID NO:1.

The variant polypeptides of the disclosure can be antibodies or Fc fusion proteins. For example but not by way of limitation, an Fc fusion proteins can be an antibody that is recombinantly expressed as a fusion protein, *e.g.,* with a cytokine protein, a toxin protein or other bioactive protein. In other aspects of the disclosure an Fc fusion protein may contain an Fc domain of an antibody, such as a variant Fc domain as disclosed herein, recombinantly expressed as a fusion protein with a fusion partner. In other aspects of the disclosure an Fc fusion protein may alternatively contain a CH2 or CH3 domain of an Fc region, such as a variant CH2 domain as disclosed herein, recombinantly expressed as a fusion protein with a fusion partner. The variant antibodies of the disclosure can be antibody-drug conjugates. For example but not by way of limitation the variant antibodies can be conjugated to mole molecule toxins or bioactive small molecule compounds. Exemplary antibodies and fusion proteins are described in Sections

The variant Fc domains of the disclosure can comprise (in addition to the one or more substitutions that give rise to increased affinity to FcγRIIB and reduced affinity to FcγRIIIA) one or more substitutions that impact effector function.

In one aspect of the disclosure the variant Fc domain may contain one or more substitutions that result in reduced binding to an FcγR and comprises an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 or 439 of the Fc domain, wherein the numbering of the residues in the Fc domain is that of the EU index as in Kabat.

For example, the variant Fc domain can contain one or more substitutions that result in reduced binding to an FcγRI and comprise an amino acid modification at any one or more of amino acid positions 238, 265, 269, 270, 327 or 329 of the Fc domain, wherein the numbering of the residues in the Fc domain is that of the EU index as in Kabat.

The variant Fc domain can contain one or more substitutions that result in reduced binding to an FcγRII and comprise an amino acid modification at any one or more of amino acid positions 238, 265, 269, 270, 292, 294, 295, 298, 303, 324, 327, 329, 333, 335, 338, 373, 376, 414, 416, 419, 435, 438 or 439 of the Fc domain, wherein the numbering of the residues in the Fc domain is that of the EU index as in Kabat.

The variant Fc domain of interest can contain one or more substitutions that result in reduced binding to an FcγRIII and comprise an amino acid modification at one or more of amino acid positions 238, 239, 248, 249, 252, 254, 265, 268, 269, 270, 272, 278, 289, 293, 294, 295, 296, 301, 303, 322, 327, 329, 338, 340, 373, 376, 382, 388, 389, 416, 434, 435 or 437 of the Fc domain, wherein the numbering of the residues in the Fc domain is that of the EU index as in Kabat.

In another aspect of the disclosure the variant Fc domain with altered FcγR binding affinity may contain one or more substitutions that result in improved binding to the FcγR and comprises an amino acid modification at any one or more of amino acid positions 255, 256, 258, 267, 268, 272, 276, 280, 283, 285, 286, 290, 298, 301, 305, 307, 309, 312, 315, 320, 322, 326, 330, 331, 333, 334, 337, 340, 360, 378, 398 or 430 of the Fc domain, wherein the numbering of the residues in the Fc domain is that of the EU index as in Kabat.

For example, the variant Fc domain can contain one or more substitutions that result in increased binding to an FcγRIII and, optionally, may further contains one or more substitutions that result in decreased binding to an FcγRII. An exemplary such variant comprises amino acid modification(s) at position(s) 298 and/or 333 of the Fc domain, wherein the numbering of the residues in the Fc domain is that of the EU index as in Kabat.

The variant Fc domain can contain one or more substitutions that result in increased binding to an FcγRII and comprise an amino acid modification at any one or more of amino acid positions 255, 256, 258, 267, 268, 272, 276, 280, 283, 285, 286, 290, 301, 305, 307, 309, 312, 315, 320, 322, 326, 330, 331, 337, 340, 378, 398 or 430 of the Fc domain, wherein the numbering of the residues in the Fc domain is that of the EU index as in Kabat. Such variant Fc domains with increased binding to an FcγRII may optionally further contains one or more substitutions that result in decreased binding to an FcγRIII and may, for example, comprise an amino acid modification at any one or more of amino acid positions 268, 272, 298, 301, 322 or 340 of the Fc domain, wherein the numbering of the residues in the Fc domain is that of the EU index as in Kabat.

In yet another aspect, the Fc variant polypeptides can be modified to increase or reduce their binding affinities to the fetal Fc receptor, FcRn, for example, by mutating the immunoglobulin constant region segment at particular regions involved in FcRn interactions (See, *e.g.,* WO 2005/123780). Accordingly, the disclosure further provides a polypeptide comprising a variant Fc domain with altered neonatal Fc receptor (FcRn) binding affinity, which polypeptide comprises an amino acid modification at any one or more of amino acid positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439 or 447 of the Fc domain, wherein the numbering of the residues in the Fc domain is that of the EU index as in Kabat. Such variant Fc domains with reduced binding to an FcRn can comprise an amino acid modification at any one or more of amino acid positions 252, 253, 254, 255, 288, 309, 386, 388, 400, 415, 433, 435, 436, 439 or 447 of the Fc domain, wherein the numbering of the residues in the Fc domain is that of the EU index as in Kabat. The above-mentioned variant Fc domains may, alternatively, contains one or more substitutions that result in increased binding to FcRn and comprise an amino acid modification at any one or more of amino acid positions 238, 256, 265, 272, 286, 303, 305, 307, 311, 312, 317, 340, 356, 360, 362, 376, 378, 380, 382, 413, 424 or 434 of the Fc domain, wherein the numbering of the residues in the Fc domain is that of the EU index as in Kabat. In yet further aspects of the disclosure the variant Fc domains can have at least one or more modification that enhances the affinity to FcRn, *e.g.,* a modification of one or more amino acid residues 251-256, 285-290, 308-314, 385-389, and 428-436 (*e.g.,* M428L), or a modification at positions 250 and 428 (*e.g.,* T250Q/M428L), see, *e.g.,* Hinton et al., 2004, J Biol Chem 279(8): 6213-6; PCT Publication No. WO 97/34631; and WO 02/060919. In particular aspects of the disclosure an antibody of the IgG class can be mutated such that at least one of amino acid residues 250, 314, and 428 of the heavy chain constant region is substituted alone, or in any combinations thereof, such as at positions 250 and 428, or at positions 250 and 314, or at positions 314 and 428, or at positions 250, 314, and 428, with positions 250 and 428 a specific combination. For position 250, the substituting amino acid residue can be any amino acid residue other than threonine, including, but not limited to, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine. For position 314, the substituting amino acid residue can be any amino acid residue other than leucine, including, but not limited to, alanine, cysteine, aspartic acid, glutamic acid, phenylalanine, glycine, histidine, isoleucine, lysine, methionine, asparagine, proline, glutamine, arginine, serine, threonine, valine, tryptophan, or tyrosine. For position 428, the substituting amino acid residues can be any amino acid residue other than methionine, including, but not limited to, alanine, cysteine, aspartic acid, glutamic acid, phenylalanine, glycine, histidine, isoleucine, lysine, leucine, asparagine, proline, glutamine, arginine, serine, threonine, valine, tryptophan, or tyrosine. Such mutations increase the antibody's binding to FcRn, which protects the antibody from degradation and increases its half-life.

Other exemplary substitutions leading to modification in Fc effector function are those disclosed in U.S. Patent No. 7,632,497. In specific embodiments, the additional substitutions are selected from those in Table 1, below. Table 1 shows the effect on binding (up, down or no change "nc") to the indicated FcγRs for the indicated substitutions (Shields et al., 2001, J Biol Chem 276(9):6591-604).

| **Table 1** | | | | | |
|---|---|---|---|---|---|
| **Substitution** | **FcRn** | **RI** | **RIIA** | **RIIB** | **RIII** |
| E233P | down | down | down | down | down |
| L234V | down | down | down | down | down |
| L235A | down | down | down | down | down |
| P238A | down | down | down | down | down |
| S239A | nc | nc | nc | nc | down |
| I253A | down | nc | nc | nc | nc |
| S254A | down | nc | nc | nc | nc |
| R255A | nc | nc | up | up | nc |
| T256A | nc | nc | up | up | up |
| E258A | nc | nc | up | up | nc |
| D265A | down | down | down | down | down |
| D265N | -- | -- | down | down | down |
| D265E | -- | -- | down | down | down |
| S267A | nc | nc | up | up | nc |
| S267G | -- | -- | nc | nc | down |
| S267T | -- | -- | down | down | down |
| H268A | nc | nc | up | up | down |
| E269A | nc | nc | nc | nc | down |
| D270A | nc | nc | down | down | down |
| D270N | -- | -- | down | down | down |
| D270E | -- | -- | down | down | nc |
| E272A | nc | nc | up | up | nc |
| N276A | nc | nc | up | up | nc |
| D280A | nc | nc | up | up | nc |
| H285A | nc | nc | up | up | nc |
| N286A | nc | nc | up | up | nc |
| K288A | down | nc | nc | nc | nc |
| K290A | nc | nc | up | up | up |
| R292A | nc | nc | down | down | nc |
| E293A | nc | nc | nc | nc | down |
| E293D | -- | -- | down | down | down |
| Q295A | nc | nc | down | down | down |
| Y296F | nc | nc | nc | nc | down |
| N297A | down | down | down | down | down |
| S298A | nc | nc | down | down | up |
| S298T | -- | -- | down | down | nc |
| S298N | -- | -- | down | down | down |
| R301A | nc | nc | up | up | down |
| R301M | -- | -- | up | up | down |
| V303A | nc | nc | nc | nc | down |
| V305A | up | nc | nc | nc | nc |
| T307A | nc | nc | up | up | nc |
| L309A | nc | nc | up | up | nc |
| Q311A | up | nc | nc | nc | nc |
| D312A | up | nc | nc | nc | nc |
| N315A | nc | nc | up | up | nc |
| K317A | up | nc | nc | nc | nc |
| K322A | nc | nc | up | up | down |
| K326A | nc | nc | up | up | nc |
| A327Q | down | down | down | down | down |
| A327S | nc | nc | down | down | down |
| A327G | nc | nc | nc | nc | down |
| P329A | down | down | down | down | down |
| P331A | nc | nc | up | up | nc |
| P331S | -- | -- | nc | down | down |
| E333A | nc | nc | nc | nc | up |
| E333Q | -- | -- | down | down | nc |
| E333N | -- | -- | down | down | down |
| E333D | -- | -- | -- | -- | up |
| K334A | nc | nc | nc | nc | up |
| K334R | -- | -- | nc | up | down |
| K334Q | -- | -- | nc | nc | up |
| K334E | -- | -- | down | nc | up |
| K334V | -- | -- | up | nc | up |
| S337A | nc | nc | up | up | nc |
| K338A | nc | nc | nc | nc | down |
| K338M | -- | -- | nc | nc | down |
| A339T | nc | nc | nc | nc | up |
| K360A | up | nc | nc | nc | nc |
| Q362A | up | nc | nc | nc | nc |
| D376A | nc | nc | nc | nc | down |
| A378Q | nc | nc | up | up | nc |
| E380A | up | nc | nc | nc | nc |
| E382A | up | nc | nc | nc | nc |
| K414A | nc | nc | down | down | nc |
| S415A | down | nc | nc | nc | nc |
| S424A | up | nc | nc | nc | nc |
| E430A | nc | nc | up | up | nc |
| H433A | down | nc | nc | nc | nc |
| N434A | up | nc | nc | nc | nc |
| H435A | down | nc | nc | nc | nc |
| Y436A | down | nc | nc | nc | nc |

In certain aspects of the disclosure the variant Fc regions of the disclosure can have one or more substitutions in their hinge regions (the portion of SEQ ID NO:1 N-terminal to the CH2 domain) that impact effector function, for example as described in WO2009/006520, particularly at the amino acid position set forth in claim 7 of WO2009/006520. In a specific aspects of the disclosure the hinge region can include at least one of the combinations of substitutions designated a through ff as set forth in claim 8 of WO2009/006520.

### 6.2. Variant Antibodies

The polypeptides of the disclosure can be antibodies comprising the variant Fc sequences described herein, referred to as "variant antibodies".

In certain aspects of the disclosure the variant antibodies of the disclosure may be monoclonal antibodies. The term "monoclonal antibody" as used herein is not limited to antibodies produced through hybridoma technology. The term "monoclonal antibody" refers to an antibody that is derived from a single clone, including any eukaryotic, prokaryotic, or phage clone and not the method by which it is produced. Monoclonal antibodies useful in connection with the present disclosure can be prepared using a wide variety of techniques known in the art including the use of hybridoma, recombinant, and phage display technologies or a combination thereof. The Fc variants of the disclosure include chimeric, primatized, humanized, or human antibodies.

The variant antibodies of the disclosure can be chimeric antibodies. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. See, *e.g.,* Morrison, 1985, Science 229(4719):1202-7; Oi et al., 1986, BioTechniques 4:214-221; Gillies et al., 1985, J Immunol Methods 125:191-202; U.S. Pat. Nos. 5,807,715; 4,816,567; and 4,816397.

The variant antibodies of the disclosure can be humanized. "Humanized" forms of non-human (*e.g.,* murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. See, *e.g.,* Riechmann et al., 1988, Nature 332:323-7; U.S. Patent Nos: 5,530,101; 5,585,089; 5,693,761; 5,693,762; and 6,180,370 to Queen et al.; EP239400; PCT publication WO 91/09967; U.S. Patent No. 5,225,539; EP592106; EP519596; Padlan, 1991, Mol Immuno, 28:489-498; Studnicka et al., 1994, Prot. Eng. 7:805-814; Roguska et al., 1994, Proc. Natl. Acad. Sci. 91:969-973; and U.S. Patent No. 5,565,332.

The variant antibodies of the disclosure can be human antibodies. Completely "human" Fc variants can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. See U.S. Patent Nos. 4,444,887 and 4,716,111; and PCT publications WO 98/46645; WO 98/50433; WO 98/24893; WO 98/16654; WO 96/34096; WO 96/33735; and WO 91/10741. Human antibodies can also be produced using transgenic mice which are incapable of expressing functional endogenous immunoglobulins but which can express human immunoglobulin genes. See, *e.g.,* PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598. In addition, companies such as Medarex (Princeton, NJ), Astellas Pharma (Deerfield, IL), Amgen (Thousand Oaks, CA) and Regeneron (Tarrytown, NY) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e.g.,* a mouse antibody, is used to guide the selection of a completely human antibody recognizing the same epitope (Jespers et al., 1988, Biotechnology 12:899-903).

The variant antibodies of the disclosure can be primatized. The term "primatized antibody" refers to an antibody comprising monkey variable regions and human constant regions. Methods for producing primatized antibodies are known in the art. See *e.g.,* U.S. Patent Nos. 5,658,570; 5,681,722; and 5,693,780.

The variant antibodies of the disclosure can be bispecific antibodies. Bispecific antibodies are monoclonal, often human or humanized, antibodies that have binding specificities for at least two different antigens. Non-limiting examples of antigen targets of bispecific antibodies include a cell-surface protein, receptor, receptor subunit, tissue-specific antigen, virally derived protein, virally encoded envelope protein, bacterially derived protein, or bacterial surface protein, *etc.*

The variant antibodies of the disclosure can be dual variable domain ("DVD") immunoglobulins ("DVD-Ig") (see, Gu & Ghayur, 2012, Methods in Enzymology 502:25-41). A DVD-Ig combines the target-binding variable domains of two monoclonal antibodies via linkers to create a tetravalent, dual-targeting single agent. Suitable linkers for use in the light chains of the DVDs of the present disclosure include those identified on Table 2.1 on page 30 of Gu & Ghayur, 2012, Methods in Enzymology 502:25-41: the short κ chain linkers ADAAP (SEQ ID NO: 22) (murine) and TVAAP (SEQ ID NO: 23) (human); the long κ chain linkers ADAAPTVSIFP (SEQ ID NO: 24) (murine) and TVAAPSVFIFPP (SEQ ID NO: 25) (human); the short λ chain linker QPKAAP (SEQ ID NO: 26) (human); the long λ chain linker QPKAAPSVTLFPP (SEQ ID NO: 27) (human); the GS-short linker GGSGG (SEQ ID NO: 28), the GS-medium linker GGSGGGGSG (SEQ ID NO: 29), and the GS-long linker GGSGGGGSGGGGS (SEQ ID NO: 30) (all GS linkers are murine and human). Suitable linkers for use in the heavy chains of the DVDs of the present disclosure include those identified on Table 2.1 on page 30 of Gu & Ghayur, 2012, Methods in Enzymology 502:25-41: the short linkers AKTTAP (SEQ ID NO: 31) (murine) and ASTKGP (SEQ ID NO: 32) (human); the long linkers AKTTAPSVYPLAP (SEQ ID NO: 33) (murine) and ASTKGPSVFPLAP (SEQ ID NO: 34) (human); the GS-short linker GGGGSG (SEQ ID NO: 35), the GS-medium linker GGGGSGGGGS (SEQ ID NO: 36), and the GS-long linker GGGGSGGGGSGGGG (SEQ ID NO: 37) (all GS linkers are murine and human). Preferably human linkers are used for human or humanized DVD-Igs.

In the present disclosure, the DVD-Ig is directed towards two different targets. The targets can be selected from EGFR, HER2, ErbB3, or any other target described in Tariq et al., U.S. Patent Application Publication No. 2011/0044980, published February 24, 2011.

Target binding domains of DVD immunoglobulins are typically arranged in tandem, with one variable domain stacked on top of another to form inner and outer Fv domains.

The variant antibodies of the disclosure include derivatized antibodies. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein (see Section 0 for a discussion of antibody conjugates), *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-natural amino acids, *e.g.,* using Ambrx technology (*See, e.g.,* Wolfson, 2006, Chem. Biol. 13(10):1011-2).

### 6.2.1. Targets of Fc Variant Antibodies

Virtually any antigen may be targeted by antibodies of the disclosure, including but not limited to proteins, subunits, domains, motifs, and/or epitopes belonging to the following list of target antigens, which includes both soluble factors such as cytokines and membrane-bound factors, including transmembrane receptors: 17-IA, 4-1BB, 4Dc, 6-keto-PGF1a, 8-iso-PGF2a, 8-oxo-dG, A1 Adenosine Receptor, A33, ACE, ACE-2, Activin, Activin A, Activin AB, Activin B, Activin C, Activin RIA, Activin RIA ALK-2, Activin RIB ALK-4, Activin RIIA, Activin RIIB, ADAM, ADAM10, ADAM12, ADAM15, ADAM17/TACE, ADAM8, ADAM9, ADAMTS, ADAMTS4, ADAMTS5, Addressins, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, Artemin, anti-Id, ASPARTIC, Atrial natriuretic factor, av/b3 integrin, Axl, b2M, B7-1, B7-2, B7-H, B-lymphocyte Stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 Osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMPs, b-NGF, BOK, Bombesin, Bone-derived neurotrophic factor, BPDE, BPDE-DNA, BTC, complement factor 3 (C3), C3a, C4, C5, C5a, C10, CA125, CAD-8, Calcitonin, cAMP, carcinoembryonic antigen (CEA), carcinoma-associated antigen, Cathepsin A, Cathepsin B, Cathepsin C/DPPI, Cathepsin D, Cathepsin E, Cathepsin H, Cathepsin L, Cathepsin O, Cathepsin S, Cathepsin V, Cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 proteins), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, *Clostridium botulinum* toxin, *Clostridium perfringens* toxin, CKb8-1, CLC, CMV, CMV UL, CNTF, CNTN-1, COX, C-Ret, CRG-2, CT-1, CTACK, CTGF, CTLA-4, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor-associated antigen, DAN, DCC, DcR3, DC-SIGN, Decay accelerating factor, des(1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, Enkephalinase, eNOS, Eot, eotaxin1, EpCAM, Ephrin B2/EphB4, EPO, ERCC, E-selectin, ET-1, Factor 10a, Factor VII, Factor VIIIc, Factor IX, fibroblast activation protein (FAP), Fas, FcR1, FEN-1, Ferritin, FGF, FGF-19, FGF-2, FGF3, FGF-8, FGFR, FGFR-3, Fibrin, FL, FLIP, Flt-3, Flt-4, Follicle stimulating hormone, Fractalkine, FZD1, FZD2, FZD3, FZD4, FZD5, FZD6, FZD7, FZD8, FZD9, FZD10, G250, Gas 6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (Myostatin), GDF-9, GDF-15 (MIC-1), GDNF, GDNF, GFAP, GFRa-1, GFR-alphal, GFR-alpha2, GFR-alpha3, GITR, Glucagon, Glut 4, glycoprotein 10b/IIIa (GP 10b/IIIa), GM-CSF, gp130, gp72, GRO, Growth hormone releasing factor, Hapten (NP-cap or NIP-cap), HB-EGF, HCC, HCMV gB envelope glycoprotein, HCMV) gH envelope glycoprotein, HCMV UL, Hemopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, High molecular weight melanoma-associated antigen (HMW-MAA), HIV gp120, HIV IIIB gp120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding proteins, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-23, interferon (INF)-alpha, INF-beta, INF-gamma, Inhibin, iNOS, Insulin A-chain, Insulin B-chain, Insulin-like growth factor 1, integrin alpha2, integrin alpha3, integrin alpha4, integrin alpha4/beta1, integrin alpha4/beta7, integrin alpha5 (alphaV), integrin alpha5/beta1, integrin alpha5/beta3, integrin alpha6, integrin beta1, integrin beta2, interferon gamma, IP-10, I-TAC, JE, Kallikrein 2, Kallikrein 5, Kallikrein 6, Kallikrein 11, Kallikrein 12, Kallikrein 14, Kallikrein 15, Kallikrein L1, Kallikrein L2, Kallikrein L3, Kallikrein L4, KC, KDR, Keratinocyte Growth Factor (KGF), laminin 5, LAMP, LAP, LAP (TGF-1), Latent TGF-1, Latent TGF-1 bp1, LBP, LDGF, LECT2, Lefty, Lewis-Y antigen, Lewis-Y related antigen, LFA-1, LFA-3, Lfo, LIF, LIGHT, lipoproteins, LIX, LKN, Lptn, L-Selectin, LT-a, LT-b, LTB4, LTBP-1, Lung surfactant, Luteinizing hormone, Lymphotoxin Beta Receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, Muellerian-inhibitin substance, Mug, MuSK, NAIP, NAP, NCAD, N-Cadherin, NCA 90, NCAM, NCAM, Neprilysin, Neurotrophin-3, -4, or -6, Neurturin, Neuronal growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, Parathyroid hormone, PARC, PARP, PBR, PBSF, PCAD, P-Cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, Proinsulin, Prorelaxin, Protein C, PS, PSA, PSCA, prostate specific membrane antigen (PSMA), PTEN, PTHrp, Ptk, PTN, R51, RANK, RANKL, RANTES, RANTES, Relaxin A-chain, Relaxin B-chain, renin, respiratory syncytial virus (RSV) F, RSV Fgp, Ret, Rheumatoid factors, RLIP76, RPA2, RSK, S100, SCF/KL, SDF-1, SERINE, Serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptors (e.g., T-cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta1, TGF-beta2, TGF-beta3, TGF-beta4, TGF-beta5, Thrombin, Thymus Ck-1, Thyroid stimulating hormone, Tie, TIMP, TIQ, Tissue Factor, TMEFF2, Tmpo, TMPRSS2, TNF, TNF-alpha, TNF-alpha beta, TNF-beta2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3M68, TR6), TNFRSF7 (CD27), TNFRSF8 (CD30), TNFRSF9 (4-1BB CD137, ILA), TNFRSF21 (DR6), TNFRSF22 (DcTRAIL R2TNFRH2), TNFRST23 (DcTRAIL R1TNFRH1), TNFRSF25 (DR3 Apo-3, LARD, TR-3, TRAMP, WSL-1), TNFSF10 (TRAIL Apo-2 Ligand, TL2), TNFSF11 (TRANCE/RANK Ligand ODF, OPG Ligand), TNFSF12 (TWEAK Apo-3 Ligand, DR3 Ligand), TNFSF13 (APRIL TALL2), TNFSF13B (BAFF BLYS, TALL1, THANK, TNFSF20), TNFSF14 (LIGHT HVEM Ligand, LTg), TNFSF15 (TL1A/VEGI), TNFSF18 (GITR Ligand AITR Ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 Ligand gp34, TXGP1), TNFSF5 (CD40 Ligand CD154, gp39, HIGM1, IMD3, TRAP), TNFSF6 (Fas Ligand Apo-1 Ligand, APT1 Ligand), TNFSF7 (CD27 Ligand CD70), TNFSF8 (CD30 Ligand CD153), TNFSF9 (4-1BB Ligand CD137 Ligand), TP-1, t-PA, Tpo, TRAIL, TRAIL R, TRAIL-R1, TRAIL-R2, TRANCE, transferring receptor, TRF, Trk, TROP-2, TSG, TSLP, tumor-associated antigen CA 125, tumor-associated antigen expressing Lewis Y related carbohydrate, TWEAK, TXB2, Ung, uPAR, uPAR-1, Urokinase, VCAM, VCAM-1, VECAD, VE-Cadherin, VE-cadherin-2, VEFGR-1 (flt-1), VEGF, VEGFR, VEGFR-3 (flt-4), VEGI, VIM, Viral antigens, VLA, VLA-1, VLA-4, VNR integrin, von Willebrands factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, and receptors for hormones and growth factors.

An antibody of the disclosure, comprising the variant Fc domains described herein, can include the CDR sequences or the variable domain sequences of a known "parent" antibody. In some embodiments, the parent antibody and the antibody of the disclosure can share similar or identical sequences except for modifications to the Fc domain as disclosed herein.

For example, a parent antibody can be substantially similar to rituximab (Rituxan®, IDEC/Genentech/Roche) (see for example U.S. Pat. No. 5,736,137), a chimeric anti-CD20 antibody approved to treat Non-Hodgkin's lymphoma; HuMax-CD20, an anti-CD20 currently being developed by Genmab, an anti-CD20 antibody described in U.S. Pat. No. 5,500,362, AME-133 (Applied Molecular Evolution), hA20 (Immunomedics, Inc.), HumaLYM (Intracel), and PRO70769 (PCT/US2003/040426, entitled "Immunoglobulin Variants and Uses Thereof"). A number of antibodies that target members of the family of epidermal growth factor receptors, including EGFR (ErbB-1), Her2/neu (ErbB-2), Her3 (ErbB-3), Her4 (ErbB-4), may benefit from the Fc polypeptides of the present invention. For example the Fc polypeptides of the present invention may find use in an antibody that is substantially similar to trastuzumab (Herceptin®, Genentech) (see for example U.S. Pat. No. 5,677,171), a humanized anti-Her2/neu antibody approved to treat breast cancer; pertuzumab (rhuMab-2C4, Omnitarg™), currently being developed by Genentech; an anti-Her2 antibody described in U.S. Pat. No. 4,753,894; cetuximab (Erbitux®, Imclone) (U.S. Pat. No. 4,943,533; PCT WO 96/40210), a chimeric anti-EGFR antibody in clinical trials for a variety of cancers; ABX-EGF (U.S. Pat. No. 6,235,883), currently being developed by Abgenix-Immunex-Amgen; HuMax-EGFr (U.S. Ser. No. 10/172,317), currently being developed by Genmab; 425, EMD55900, EMD62000, and EMD72000 (Merck KGaA) (U.S. Pat. No. 5,558,864; Murthy et al. 1987, Arch Biochem Biophys. 252(2):549-60; Rodeck et al., 1987, J Cell Biochem. 35(4):315-20; Kettleborough et al., 1991, Protein Eng. 4(7):773-83); ICR62 (Institute of Cancer Research) (PCT WO 95/20045; Modjtahedi et al., 1993, J Cell Biophys. 1993, 22(1-3):129-46; Modjtahedi et al., 1993, Br J Cancer. 1993, 67(2):247-53; Modjtahedi et al., 1996, Br J Cancer, 73(2):228-35; Modjtahedi et al, 2003, Int J Cancer, 105(2):273-80); TheraCIM hR3 (YM Biosciences, Canada and Centro de Immunologia Molecular, Cuba (U.S. Pat. No. 5,891,996; U.S. Pat. No. 6,506,883; Mateo et al, 1997, Immunotechnology, 3(1):71-81); mAb-806 (Ludwig Institute for Cancer Research, Memorial Sloan-Kettering) (Jungbluth et al. 2003, Proc Natl Acad Sci USA. 100(2):639-44); KSB-102 (KS Biomedix); MR1-1 (IVAX, National Cancer Institute) (PCT WO 0162931A2); and SC100 (Scancell) (PCT WO 01/88138). In another preferred aspect of the disclosure the Fc polypeptides of the present invention may find use in alemtuzumab (Campath®, Millenium), a humanized monoclonal antibody currently approved for treatment of B-cell chronic lymphocytic leukemia. The Fc polypeptides of the present invention may find use in a variety of antibodies or Fc fusions that are substantially similar to other clinical products and candidates, including but not limited to muromonab-CD3 (Orthoclone OKT3®), an anti-CD3 antibody developed by Ortho Biotech/Johnson & Johnson, ibritumomab tiuxetan (Zevalin®), an anti-CD20 antibody developed by IDEC/Schering AG, gemtuzumab ozogamicin (Mylotarg®), an anti-CD33 (p67 protein) antibody developed by Celltech/Wyeth, abciximab (ReoPro®), developed by Centocor/Lilly, basiliximab (Simulect®), developed by Novartis, palivizumab (Synagis®), developed by MedImmune, infliximab (Remicade®), an anti-TNFalpha antibody developed by Centocor, adalimumab (Humira®), an anti-TNFalpha antibody developed by Abbott, Humicade™ an anti-TNFalpha antibody developed by Celltech, ABX-CBL, an anti-CD 147 antibody being developed by Abgenix, ABX-IL8, an anti-IL8 antibody being developed by Abgenix, ABX-MA1, an anti-MUC18 antibody being developed by Abgenix, Pemtumomab (R1549, 90Y-muHMFG1), an anti-MUCl In development by Antisoma, Therex (R1550), an anti-MUC1 antibody being developed by Antisoma, AngioMab (AS 1405), being developed by Antisoma, HuBC-1, being developed by Antisoma, Thioplatin (AS1407) being developed by Antisoma, Antegren® (natalizumab), an anti-alpha-4-beta-1 (VLA-4) and alpha-4-beta-7 antibody being developed by Biogen, VLA-1 mAb, an anti-VLA-1 integrin antibody being developed by Biogen, LTBR mAb, an anti-lymphotoxin beta receptor (LTBR) antibody being developed by Biogen, CAT-152, an anti-TGF-β2 antibody being developed by Cambridge Antibody Technology, J695, an anti-IL-12 antibody being developed by Cambridge Antibody Technology and Abbott, CAT-192, an anti-TGFβ1 antibody being developed by Cambridge Antibody Technology and Genzyme, CAT-213, an anti-Eotaxinl antibody being developed by Cambridge Antibody Technology, LymphoStat-B™ an anti-Blys antibody being developed by Cambridge Antibody Technology and Human Genome Sciences Inc., TRAIL-R1 mAb, an anti-TRAIL-Rl antibody being developed by Cambridge Antibody Technology and Human Genome Sciences, Inc., Avastin™ (bevacizumab, rhuMAb-VEGF), an anti-VEGF antibody being developed by Genentech, an anti-HER receptor family antibody being developed by Genentech, Anti-Tissue Factor (ATF), an anti-Tissue Factor antibody being developed by Genentech, Xolair™ (Omalizumab), an anti-IgE antibody being developed by Genentech, Raptiva™ (Efalizumab), an anti-CD11a antibody being developed by Genentech and Xoma, MLN-02 Antibody (formerly LDP-02), being developed by Genentech and Millenium Pharmaceuticals, HuMax CD4, an anti-CD4 antibody being developed by Genmab, HuMax-IL15, an anti-IL15 antibody being developed by Genmab and Amgen, HuMax-Inflam, being developed by Genmab and Medarex, HuMax-Cancer, an anti-Heparanase I antibody being developed by Genmab and Medarex and Oxford GcoSciences, HuMax-Lymphoma, being developed by Genmab and Amgen, HuMax-TAC, being developed by Genmab, IDEC-131, and anti-CD40L antibody being developed by IDEC Pharmaceuticals, IDEC-151 (Clenoliximab), an anti-CD4 antibody being developed by IDEC Pharmaceuticals, IDEC-114, an anti-CD80 antibody being developed by IDEC Pharmaceuticals, IDEC-152, an anti-CD23 being developed by IDEC Pharmaceuticals, anti-macrophage migration factor (MIF) antibodies being developed by IDEC Pharmaceuticals, BEC2, an anti-idiotypic antibody being developed by Imclone, IMC-1C11, an anti-KDR antibody being developed by Imclone, DC101, an anti-flk-1 antibody being developed by Imclone, anti-VE cadherin antibodies being developed by Imclone, CEA-Cide™ (labetuzumab), an anti-carcinoembryonic antigen (CEA) antibody being developed by Immunomedics, LymphoCide™ (Epratuzumab), an anti-CD22 antibody being developed by Immunomedics, AFP-Cide, being developed by Immunomedics, MyelomaCide, being developed by Immunomedics, LkoCide, being developed by Immunomedics, ProstaCide, being developed by Immunomedics, MDX-010, an anti-CTLA4 antibody being developed by Medarex, MDX-060, an anti-CD30 antibody being developed by Medarex, MDX-070 being developed by Medarex, MDX-018 being developed by Medarex, Osidem™ (IDM-1), and anti-Her2 antibody being developed by Medarex and Immuno-Designed Molecules, HuMax™-CD4, an anti-CD4 antibody being developed by Medarex and Genmab, HuMax-IL15, an anti-IL15 antibody being developed by Medarex and Genmab, CNTO 148, an anti-TNFa antibody being developed by Medarex and Centocor/J&J, CNTO 1275, an anti-cytokine antibody being developed by Centocor/J&J, MOR101 and MOR102, anti-intercellular adhesion molecule-1 (ICAM-1) (CD54) antibodies being developed by MorphoSys, MOR201, an anti-fibroblast growth factor receptor 3 (FGFR-3) antibody being developed by MorphoSys, Nuvion® (visilizumab), an anti-CD3 antibody being developed by Protein Design Labs, HuZAF™, an anti-gamma interferon antibody being developed by Protein Design Labs, Anti-α5β1 Integrin, being developed by Protein Design Labs, anti-IL-12, being developed by Protein Design Labs, ING-1, an anti-Ep-CAM antibody being developed by Xoma, and MLN01, an anti-Beta2 integrin antibody being developed by Xoma.

In one aspect of the disclosure the variants of the present invention may be used for the treatment of autoimmune, inflammatory, or transplant indications. Target antigens and clinical products and candidates that are relevant for such diseases include but are not limited to anti-α4β7 integrin antibodies such as LDP-02, anti-beta2 integrin antibodies such as LDP-01, anti-complement (C5) antibodies such as 5G1.1, anti-CD2 antibodies such as BTI-322, MEDI-507, anti-CD3 antibodies such as OKT3, SMART anti-CD3, anti-CD4 antibodies such as IDEC-151, MDX-CD4, OKT4A, anti-CD11a antibodies, anti-CD 14 antibodies such as IC14, anti-CD 18 antibodies, anti-CD23 antibodies such as IDEC 152, anti-CD25 antibodies such as Zenapax, anti-CD40L antibodies such as 5c8, Antova, IDEC-131, anti-CD64 antibodies such as MDX-33, anti-CD80 antibodies such as IDEC-114, anti-CD 147 antibodies such as ABX-CBL, anti-E-selectin antibodies such as CDP850, anti-gpIIb/IIIa antibodies such as ReoPro/Abcixima, anti-ICAM-3 antibodies such as ICM3, anti-ICE antibodies such as VX-740, anti-FcRl antibodies such as MDX-33, anti-IgE antibodies such as rhuMab-E25, anti-IL-4 antibodies such as SB-240683, anti-IL-5 antibodies such as SB-240563, SCH55700, anti-IL-8 antibodies such as ABX-IL8, anti-interferon gamma antibodies, anti-TNF (TNF, TNFa, TNFa, TNF-alpha) antibodies such as CDP571, CDP870, D2E7, Infliximab, MAK-195F, and anti-VLA-4 antibodies such as Antegren.

Exemplary antibodies which can be engineered to incorporated the variant Fc regions of the disclosure are set forth in Table 2 below:

| **Table 2** | | | |
|---|---|---|---|
| **Antibody target & name** | **Chain** | **Sequence Identifier** | **Sequence** |
| Anti-CD40 (S2C6) | VL | SEQ ID NO:3 | |
| | VH | SEQ ID NO:4 | |
| Anti-CD20 (rituximab) | VL | SEQ ID NO:5 | |
| | VH | SEQ ID NO:6 | |
| Anti-CD25 (daclizumab) | VL | SEQ ID NO:7 | |
| | VH | SEQ ID NO: 8 | |
| Anti-CD25 (basiliximab) | VL | SEQ ID NO:9 | |
| | VH | SEQ ID NO:10 | |
| Anti-TNFa (adalimumab) | VL | SEQ ID NO:11 | |
| | VH | SEQ ID NO:12 | |
| Anti-TNFa (infliximab) | VL | SEQ ID NO:13 | |
| | VH | SEQ ID NO:14 | |
| Anti-IL-6R (tocilizumab) | VL | SEQ ID NO:15 | |
| | | | |
| | VH | SEQ ID NO:16 | |
| Anti-IL-1 (canakinumab) | VL | SEQ ID NO:17 | |
| | VH | SEQ ID NO:18 | |
| Anti-BAFF (belimumab) | VL | SEQ ID NO:19 | |
| | VH | SEQ ID NO:20 | |

Several of the antibodies described in this section have been subject to mutational analysis to improve their biological properties. Such mutant antibodies having desirable properties can be modified to incorporate the variant CH2 domains and Fc regions of the disclosure. US 2010/0266613 A1, for example, discloses variant V_{L} and V_{H} sequences of the anti-TNFa antibody adalimumab. The variant CH2 domains and Fc regions of the disclosure can be incorporated into any of the variant anti-TNFα antibodies disclosed in US 2010/0266613 A1. In some aspects of the disclosure the variant anti-TNFα antibody can comprise one of more of the substitutions in Table 5 of US 2010/0266613, *i.e.,* A25W, Q27R, Q27T, I29V, R30Q, and L33E in the V_{L} chain. In other aspects of the disclosure the variant anti-TNFa antibody can comprise a combination of substitutions from Table 10 of US 2010/0266613, *i.e.,* 129T/A34G, N31T/A34G, R30Q/A34S, R30Q, Q27G/A34G, Q27H/A34S, Q27R/A34S, G28S/A34S, N31T/A34S, or N31S/A34S in the V_{L} chain, most preferably G28S/A34S. The stretch of amino acids spanning A25 through A34 is in bold, underlined font in Table 2 above.

In some aspects of the disclosure antibodies against infectious diseases may be used. Antibodies against eukaryotic cells include antibodies targeting yeast cells, including but not limited to *Saccharomyces cerevisiae, Hansenula polymorpha, Kluyveromyces fragilis* and *K*. *lactis, Pichia guillerimondii* and *P. pastoris, Schizosaccharomyces pombe, Plasmodium falciparium,* and *Yarrowia lipolytica.*

Antibodies against additional fungal cells are also useful, including target antigens associated with Candida strains including Candida glabrata, Candida albicans, C. krusei, C. lusitaniae and C. maltosa, as well as species of Aspergillus, Cryptococcus, Histoplasma, Coccidioides, Blastomyces, and Penicillium, among others.

Antibodies directed against target antigens associated with protozoa include, but are not limited to, antibodies associated with *Trypanosoma, Leishmania* species including *Leishmania donovanii; Plasmodium* spp., *Pneumocystis carinii, Cryptosporidium parvum, Giardia lamblia, Entamoeba histolytica,* and *Cyclospora cayetanensis.*

Antibodies against prokaryotic antigens are also useful, including antibodies against suitable bacteria such as pathogenic and non-pathogenic prokaryotes including but not limited to Bacillus, including Bacillus anthracis; Vibrio, e.g. V. cholerae; Escherichia, e.g. Enterotoxigenic E. coli, Shigella, e.g. S. dysenteriae; Salmonella, e.g. S. typhi; Mycobacterium e.g. M. tuberculosis, M. leprae; Clostridium, e.g. C. botulinum, C. tetani, C. difficile, C. perfringens; Cornyebacterium, e.g. C. diphtheriae; Streptococcus, S. pyogenes, S. pneumoniae; Staphylococcus, e.g. S. aureus; Haemophilus, e.g. H. influenzae; Neisseria, e.g. N. meningitidis, N. gonorrhoeae; Yersinia, e.g. Y. lamblia, Y. pestis, Pseudomonas, e.g. P. aeruginosa, P. putida; Chlamydia, e.g. C. trachomatis; Bordetella, e.g. B. pertussis; Treponema, e.g. T. palladium; B. anthracis, Y. pestis, Brucella spp., F. tularensis, B. mallei, B. pseudomallei, B. mallei, B. pseudomallei, C. botulinum, Salmonella spp., SEB V. cholerae toxin B, E. coli 0157:H7, Listeria spp., Trichosporon beigelii, Rhodotorula species, Hansenula anomala, Enterobacter sp., Klebsiella sp., Listeria sp., Mycoplasma sp. and the like.

In some aspects, the antibodies are directed against viral infections; these viruses include, but are not limited to, including orthomyxoviruses, (e.g. influenza virus), paramyxoviruses (e.g respiratory syncytial virus, mumps virus, measles virus), adenoviruses, rhinoviruses, coronaviruses, reoviruses, togaviruses (e.g. rubella virus), parvoviruses, poxviruses (e.g. variola virus, vaccinia virus), enteroviruses (e.g. poliovirus, coxsackievirus), hepatitis viruses (including A, B and C), herpesviruses (e.g. Herpes simplex virus, varicella-zoster virus, cytomegalovirus, Epstein-Barr virus), rotaviruses, Norwalk viruses, hantavirus, arenavirus, rhabdovirus (e.g. rabies virus), retroviruses (including HIV, HTLV-I and -II), papovaviruses (e.g. papillomavirus), polyomaviruses, and picornaviruses, and the like.

### 6.3. Fc Fusion Proteins

In one aspect of the disclosure the polypeptides of the invention may be Fc fusion proteins. Fc-based fusion proteins are typically composed of an immunoglobin Fc domain that is directly linked to another peptide. As explained by Czajkowsky et al., 2012, EMBO Mol Med 4:1015-1028, the fusion partner can be any other proteinaceous molecule of interest, such as a ligand that activates upon interaction with a cell-surface receptor, a peptidic antigen (Ag) against a challenging pathogen or a 'bait' protein to identify binding partners assembled in a protein microarray. Most frequently, an Fc domain is fused to a polypeptide with therapeutic potential to endow the fusion with a number of additional beneficial biological and pharmacological properties. The presence of an Fc domain can markedly increase a protein's plasma half life, which prolongs its therapeutic activity owing to its interaction with the salvage neonatal Fc-receptor (FcRn; Roopenian & Akilesh, 2007, Nat Rev Immunol 7: 715-725), as well as to the slower renal clearance for larger sized molecules (Kontermann, 2011, Curr Opin Biotechnol 22: 868-876). The attached Fc domain also enables these molecules to interact with Fc-receptors (FcRs) found on immune cells (Nimmerjahn & Ravetch, 2008, Nat Rev Immunol 8: 34-47).

Accordingly, an Fc fusion combines the Fc region of an antibody, and thus its favorable effector functions and pharmacokinetics, with the target-binding region of a receptor, ligand, or some other protein or protein domain. The role of the latter is to mediate target recognition, and thus it is functionally analogous to the antibody variable region. Because of the structural and functional overlap of Fc fusions with antibodies, the discussion on antibodies in the present disclosure extends to Fc fusions unless indicated otherwise.

In exemplary aspects of the disclosure the Fc fusion partner can be the extracellular domain ("ECD") of TNF receptor II; the first ECD of lymphocyte function-associated antigen 3 (LFA-3); the ECD of human cytotoxic T lymphocyte associated molecule-4 (CTLA-4); the C-terminus of the IL-1R accessory protein ligand binding region fused to the N-terminus of the IL-1RI ECD; peptide thrombopoietin (TPO) mimetic; ECD of CTLA-4 with the two amino acid substitutions L104E and A29Y; or ECDs of VEGF receptors 1 and 2.

An Fc fusion protein of the disclosure, comprising the variant Fc domains described herein, can based based on a known "parent" Fc fusion, such as the approved biologics described in Table 3.

| **Table 3** | | | |
|---|---|---|---|
| **International non-proprietary (trade) name** | **Description** | **Mode of action** | **Year & Indication of first US approval** |
| Etanercept (Enbrel®) | 75 kDa soluble extracellular domain (ECD) of tumor necrosis factor (TNF) receptor II fused to human IgG1 Fc | Binds membrane-bound and soluble forms of TNF, thereby reducing concentrations of inflammatory cytokines | 1998 |
| | | | Rheumatoid arthritis |
| Alefacept (Amevive®) | First ECD of lymphocyte function-associated antigen 3 (LFA-3) fused to human IgG1 Fc | Binds CD2; blocks the interactions between LFA on APCs with CD2 on T cells, thereby inhibiting T-cell activation | 2003 |
| | | | Plaque psoriasis |
| Abatacept (Orencia®) | ECD of human cytotoxic T lymphocyte associated molecule-4 (CTLA-4) fused to human IgG1 Fc | Blocks the interactions between CD80 or CD86 on APCs and CD28 on T cells, thereby inhibiting T-cell activation | 2005 |
| | | | Rheumatoid arthritis |
| Rilonacept (Arcalyst®) | Two chains, each comprising the C-terminus of the IL-1R accessory protein ligand binding region fused to the N-terminus of the IL-1RI ECD, fused to human IgG1 Fc | Binds IL-1, thereby preventing interaction with endogenous cell-surface receptors | 2008 |
| | | | Plaque psoriasis |
| Romiplostim (Nplate®) | Peptide thrombopoietin (TPO) mimetic fused to the C-terminus of aglycosylated human IgG1 Fc; produced in *E. Coli* | Binds and agonizes the TPO receptor; Fc functionality minimized due to lack of glycosylation | 2008 |
| | | | Thrombocytopenia |
| Belatacept (Nulojix®) | ECD of CTLA-4 fused to human IgG1 Fc; differs from abatacept by two amino acid substitutions (L104E, A29Y) in the CTLA-4 region | Blocks the interactions between CD80 or CD86 on APCs and CD28 on T cells, thereby inhibiting T-cell activation | 2011 |
| | | | Prophylaxis of organ rejection in adult kidney transplant recipients |
| Aflibercept (Eylea™) | ECDs of VEGF receptors 1 and 2 fused to human IgG1 Fc | Binds all forms of VEGF-A, as well as placental growth factor, thereby inhibiting angiogenesis | 2011 |
| | | | Wet age-related macular degeneration |

In some aspects of the disclosure the parent Fc fusion and the Fc fusion of the disclosure can share similar or identical sequences except for modifications to the Fc domain as disclosed herein.

Fc fusion proteins can also contain just a variant CH2 domain instead of a whole Fc region. Fusion proteins containing a variant CH2 domain can be used, for example, as a dimerization domain and/or to direct the fusion polypeptide to FCγIIB. In one aspect of the disclosure the fusion partner can be another Fc domain, such as an IgE Fc domain, creating a "tandem" Fc polypeptide. An IgG-IgE fusion polypeptide was shown to binds FcεR and FcγRIIB and shut down mast cell degranulation. See Cermerski et al., 2012, Immunol. Lett. 143:34-43

### 6.4. Nucleic Acids and Expression Systems

The present disclosure encompasses nucleic acid molecules and host cells encoding the Fc variant polypeptides of the disclosure.

A variant antibody of the disclosure that is an antibody can be prepared by recombinant expression of immunoglobulin light and heavy chain genes in a host cell. For example, to express an antibody recombinantly, a host cell is transfected with one or more recombinant expression vectors carrying DNA fragments encoding the immunoglobulin light and heavy chains of the antibody such that the light and heavy chains are expressed in the host cell and, optionally, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397.

In one aspect of the disclosure the Fc variant polypeptides may be similar to their wild-type equivalents but for changes in their Fc domains. To generate nucleic acids encoding such Fc variant polypeptides, a DNA fragment encoding the Fc domain or a portion of the Fc domain of the wild-type antibody (referred to as the "wild-type Fc domain") can be synthesized and used as a template for mutagenesis to generate a polypeptide as described herein using routine mutagenesis techniques; alternatively, a DNA fragment encoding the polypeptide can be directly synthesized.

Once DNA fragments encoding wild-type Fc domains are obtained, these DNA fragments can be further manipulated by standard recombinant DNA techniques, for example, to convert the constant region genes to full-length antibody chain genes. In these manipulations, a CH-encoding DNA fragment is operatively linked to another DNA fragment encoding another protein, such as an antibody variable region or a flexible linker. The term "operatively linked," as used in this context, is intended to mean that the two DNA fragments are joined such that the amino acid sequences encoded by the two DNA fragments remain in-frame.

To express the Fc variant polypeptides of the disclosure, DNAs encoding partial or full-length light and heavy chains, obtained as described above, are inserted into expression vectors such that the genes are operatively linked to transcriptional and translational control sequences. In this context, the term "operatively linked" is intended to mean that a polypeptide gene is ligated into a vector such that transcriptional and translational control sequences within the vector serve their intended function of regulating the transcription and translation of the polypeptide gene. The expression vector and expression control sequences are chosen to be compatible with the expression host cell used. A variant antibody light chain gene and the antibody heavy chain gene can be inserted into separate vectors or, more typically, both genes are inserted into the same expression vector.

The polypeptide genes are inserted into the expression vector by standard methods (e.g., ligation of complementary restriction sites on the polypeptide gene fragment and vector, or blunt end ligation if no restriction sites are present). Prior to insertion of the variant Fc domain sequences, the expression vector can already carry antibody variable region sequences. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, the recombinant expression vectors of the disclosure carry regulatory sequences that control the expression of the antibody chain genes in a host cell. The term "regulatory sequence" is intended to include promoters, enhancers and other expression control elements (*e.g.,* polyadenylation signals) that control the transcription or translation of the antibody chain genes. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, *etc.* Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.,* the adenovirus major late promoter (AdMLP)) and polyoma. For further description of viral regulatory elements, and sequences thereof, see, *e.g.,* U.S. Patent No. 5,168,062 by Stinski, U.S. Patent No. 4,510,245 by Bell et al.*,* and U.S. Patent No. 4,968,615 by Schaffner et al.

In addition to the antibody chain genes and regulatory sequences, the recombinant expression vectors of the disclosure can carry additional sequences, such as sequences that regulate replication of the vector in host cells (*e.g.,* origins of replication) and selectable marker genes. The selectable marker gene facilitates selection of host cells into which the vector has been introduced (*See, e.g.,* U.S. Patents Nos. 4,399,216, 4,634,665 and 5,179,017, all by Axel et al.). For example, typically the selectable marker gene confers resistance to drugs, such as G418, puromycin, blasticidin, hygromycin or methotrexate, on a host cell into which the vector has been introduced. Suitable selectable marker genes include the dihydrofolate reductase (DHFR) gene (for use in DHFR⁻ host cells with methotrexate selection/amplification) and the neo gene (for G418 selection). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, lipofection, calcium-phosphate precipitation, DEAE- dextran transfection and the like.

It is possible to express the polypeptides of the disclosure in either prokaryotic or eukaryotic host cells. In certain embodiments, expression of polypeptides is performed in eukaryotic cells, *e.g.,* mammalian host cells, for optimal secretion of a properly folded and immunologically active polypeptide. Exemplary mammalian host cells for expressing the recombinant polypeptides of the disclosure include Chinese Hamster Ovary (CHO cells) (including DHFR⁻ CHO cells, described in Urlaub and Chasin, 1980, Proc. Natl. Acad. Sci. USA 77:4216-4220, used with a DHFR selectable marker, *e.g.,* as described in Kaufman and Sharp, 1982, Mol. Biol. 159:601-621), NS0 myeloma cells, COS cells, 293 cells and SP2/0 cells. When recombinant expression vectors encoding polypeptide genes are introduced into mammalian host cells, the polypeptides are produced by culturing the host cells for a period of time sufficient to allow for expression of the polypeptide in the host cells or secretion of the polypeptide into the culture medium in which the host cells are grown. Polypeptides can be recovered from the culture medium using standard protein purification methods. Host cells can also be used to produce portions of intact polypeptides, such as Fab fragments or scFv molecules. It is understood that variations on the above procedure are within the scope of the present disclosure.

Recombinant DNA technology can also be used to remove some or all of the DNA encoding either or both of the light and heavy chains that is not necessary for binding to antigen. The molecules expressed from such truncated DNA molecules are also encompassed by the polypeptides of the disclosure.

In some aspects of the disclosure polypeptides of the disclosure can be bifunctional antibodies. Such antibodies, in which one heavy and one light chain are specific for one antigen and the other heavy and light chain are specific for a second antigen, can be produced by crosslinking an antibody of the disclosure to a second antibody by standard chemical crosslinking methods. Bifunctional antibodies can also be made by expressing a nucleic acid engineered to encode a bifunctional antibody.

In certain aspects of the disclosure dual specific antibodies, i.e. antibodies that bind one antigen and a second, unrelated antigen using the same binding site, can be produced by mutating amino acid residues in the light chain and/or heavy chain CDRs. Exemplary second antigens include a proinflammatory cytokine (such as, for example, lymphotoxin, interferon-γ, or interleukin-1). Dual specific polypeptides can be produced, *e.g.,* by mutating amino acid residues in the periphery of the antigen binding site (*See, e.g.,* Bostrom et al., 2009, Science 323:1610-1614). Dual functional polypeptides can be made by expressing a nucleic acid engineered to encode a dual specific polypeptide.

Polypeptides of the disclosure can also be produced by chemical synthesis (*e.g.,* by the methods described in Solid Phase Peptide Synthesis, 2nd ed., 1984 The Pierce Chemical Co., Rockford, Ill.). Polypeptides can also be generated using a cell-free platform (see, *e.g.,* Chu et al., Biochemia No. 2, 2001 (Roche Molecular Biologicals)).

Methods for recombinant expression of Fc fusion proteins are described in Flanagan et al., Methods in Molecular Biology, vol. 378: Monoclonal Antibodies: Methods and Protocols.

Once a polypeptide of the disclosure has been produced by recombinant expression, it can be purified by any method known in the art for purification of an immunoglobulin molecule, for example, by chromatography (*e.g.,* ion exchange, affinity, particularly by affinity for antigen after Protein A or Protein G selection, and sizing column chromatography), centrifugation, differential solubility, or by any other standard technique for the purification of proteins. Further, the polypeptides of the present disclosure or fragments thereof can be fused to heterologous polypeptide sequences described herein or otherwise known in the art to facilitate purification.

Once isolated, a polypeptide can, if desired, be further purified, *e.g.,* by high performance liquid chromatography (See, *e.g.,* Fisher, Laboratory Techniques In Biochemistry And Molecular Biology (Work and Burdon, eds., Elsevier, 1980)), or by gel filtration chromatography on a Superdex™ 75 column (Pharmacia Biotech AB, Uppsala, Sweden).

### 6.5. Biological Activity of Fc Variant Polypeptides

Due to the incorporation of amino acid substitutions in the Fc region that impact binding to FcγRIIIA and/or FcγRIIB, the polypeptides of the disclosure display modified biological activity, e.g., modified effector function and/or binding to FcγRIIIA and/or FcγRIIB.

In one aspect of the disclosure the effector function may be ADCC. Accordingly, the disclosure provides variant Fc polypeptide that are characterized by exhibiting ADCC that is reduced by at least about 30%, at least about 40%, at least about 50%, at least about 60%, at least about 70% or even more as compared to a non-variant Fc polypeptide, *i.e.,* a polypeptide that is identical but for the substitution(s) that increase binding to FcγRIIB and/or decrease binding to FcyRIIIA, for example one or more of the substitutions V263L, V266L, V273C, V273E, V273F, V273L, V273M, V273S, V273Y, V305K, and V305W.

In certain aspects of the disclosure the reduction in ADCC can be measured in an *in vitro* assay at a polypeptide concentration of 1 µg/mL or 2 µg/mL or more (*e.g.,* 3 µg/mL, 4 µg/mL or 5 µg/mL) using an effector to target cell ratio of, for example, 25:1, 40:1, 50:1 or 60:1, for example when using PBMC effector cells from 3 or more, 6 or more, 10 or more, or 50 or more healthy donors. ADCC activity can be measured by flow cytometry, as described in Example 9, or by measuring ⁵¹Chromium release, as described in Example 10. The target cell utilized in an ADCC assay will depend on the binding specificity of the variant polypeptide, and can be readily determined by one of skill in the art. For example, as described in Example 9, Raji cells are suitable target cells for assaying ADCC activity of antibody Hu1D10 (and Fc variants thereof) and, as described in Example 10, Lymphoma RL cells are suitable target cells for assaying ADCC activity of an anti-CD40 antibody (and Fc variants thereof).

In another aspect of the disclosure the effector function can be immune activation of a target cell by a cross-linking Fc polypeptide. For example in one assay, the target cell is a dendritic cell and the the cross-linking Fc polypeptide is an anti-CD40 antibody. Typically, binding of an Fc region to FcγRIIB provides a negative signal to FcγRIIB positive cells via the receptor's ITAM motif. However, binding of an Fc region of an anti-CD40 antibody to FcγRIIB on the surface of dendritic cells improves crosslinking of CD40, resulting in increased IL-12 production by the dendritic cells. Increasing affinity to FcγRIIB therefore results in higher IL-12 secretion. Accordingly, the disclosure provides variant Fc polypeptide whose Fc region increases IL-12 secretion in dendritic cells when grafted onto a CD40 antibody. In various aspects of the disclosure the Fc region can activate dendritic cells by at least about 10%, at least about 15%, at least about 20%, at least about 25%, at least 30% , at least about 35%, at least about 40%, or even more as compared to a non-variant anti-CD40 antibody, *i.e.,* an anti-CD40 antibody that is identical but for the substitution(s) that increase binding to FcγRIIB and optionally also decrease binding to FcγRIIIA, for example one or more of the substitutions V263L, V266L, V273C, V273E, V273F, V273L, V273M, V273S, V273Y, V305K, and V305W.

In certain aspects of the disclosure the immune activation of dendritic cells can be measured in an IL-12p70 secretion assay. Briefly, monocyte-derived immature dendritic cells ("moDC") can be stimulated with a polypeptide of the disclosure and primed with IFNγ, and the resulting amount of IL-12p70 produced assayed, for example by ELISA. In an exemplary aspect of the disclosure the IL-12p70 secretion assay can be performed as described in Example 11 below.

In yet other aspects of the disclosure a variant polypeptide of the disclosure may display increased binding to FcγRIIB and/or reduced binding to FcγRIIIA. Exemplary binding assays are described in Examples 7 and 8. In certain aspects of the disclosure the binding of a variant polypeptide to FcγRIIB may be at least about 10%, at least about 20%, by at least about 30%, at least about 40%, or at least about 50% greater than the binding to FcγRIIB of a non-variant Fc polypeptide, *e.g.,* a polypeptide that is identifical but for the one or more of the substitutions of V263L, V266L, V273C, V273E, V273F, V273L, V273M, V273S, V273Y, V305K, and V305W. In further aspects of the disclosure the binding of a variant polypeptide to FcγRIIIA may be at least about 10%, at least about 20%, by at least about 30%, at least about 40%, or at least about 50% less than the binding to FcγRIIIA of a non-variant Fc polypeptide, *e.g.,* a polypeptide that is identifical but for the one or more of the substitutions of V263L, V266L, V273C, V273E, V273F, V273L, V273M, V273S, V273Y, V305K, and V305W.

### 6.6. Polypeptide Conjugates

The polypeptides of the disclosure include polypeptide conjugates that are modified, *e.g.,* by the covalent attachment of any type of molecule to the polypeptide, such that covalent attachment does not interfere with binding to antigen.

In certain aspects, a polypeptide of the disclosure can be conjugated to an effector moiety or a label. The term "effector moiety" as used herein includes, for example, antineoplastic agents, drugs, toxins, biologically active proteins, for example enzymes, antibody or antibody fragments, synthetic or naturally occurring polymers, nucleic acids (*e.g.,* DNA and RNA), radionuclides, particularly radioiodide, radioisotopes, chelated metals, nanoparticles and reporter groups such as fluorescent compounds or compounds which can be detected by NMR or ESR spectroscopy.

In one example, polypeptides can be conjugated to an effector moiety, such as a cytotoxic agent, a radionuclide or drug moiety to modify a given biological response. The effector moiety can be a protein or polypeptide, such as, for example and without limitation, a toxin (such as abrin, ricin A, *Pseudomonas* exotoxin, or *Diphtheria* toxin), a signaling molecule (such as α-interferon, β-interferon, nerve growth factor, platelet derived growth factor or tissue plasminogen activator), a thrombotic agent or an anti-angiogenic agent (*e.g.,* angiostatin or endostatin) or a biological response modifier such as a cytokine or growth factor (*e.g.,* interleukin-1 (IL-I), interleukin-2 (IL-2), interleukin-6 (IL-6), granulocyte macrophage colony stimulating factor (GM-CSF), granulocyte colony stimulating factor (G-CSF), or nerve growth factor (NGF)).

In another example the effector moieties can be cytotoxins or cytotoxic agents. Examples of cytotoxins and cytotoxic agents include taxol, cytochalasin B, gramicidin D, ethidium bromide, emetine, mitomycin, etoposide, tenoposide, vincristine, vinblastine, colchicin, doxorubicin, daunorabicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector moieties also include, but are not limited to, antimetabolites (*e.g.* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BSNU) and lomustine (CCNU), cyclothosphamide, busulfan, dibromomannitol, streptozotocin, mitomycin C5 and cis-dichlorodiamine platinum (II) (DDP) cisplatin), anthracyclines (*e.g.,* daunorubicin (formerly daunomycin) and doxorubicin), antibiotics (*e.g.,* dactinomycin (formerly actinomycin), bleomycin, mithramycin, anthramycin (AMC), calicheamicins or duocarmycins), and anti-mitotic agents (e.g., vincristine and vinblastine).

Other effector moieties can include radionuclides such as, but not limited to, ¹¹¹In and ⁹⁰Y, Lu¹⁷⁷, Bismuth²¹³, Californium²⁵², Iridium¹⁹² and Tungsten¹⁸⁸/Rhenium¹⁸⁸ and drugs such as, but not limited to, alkylphosphocholines, topoisomerase I inhibitors, taxoids and suramin.

Techniques for conjugating such effector moieties to polypeptides are well known in the art (See, *e.g.,* Hellstrom et al., Controlled Drug Delivery, 2nd Ed., at pp. 623-53 (Robinson et al., eds., 1987)); Thorpe et al., 1982, Immunol. Rev. 62:119-58 and Dubowchik et al., 1999, Pharmacology and Therapeutics 83:67-123).

In one example, the polypeptide is fused via a covalent bond (*e.g.,* a peptide bond), through the polypeptide's N-terminus or the C-terminus or internally, to an amino acid sequence of another protein (or portion thereof; for example, at least a 10, 20 or 50 amino acid portion of the protein). The polypeptide can linked to the other protein at the N-terminus of the Fc domain of the polypeptide. Recombinant DNA procedures can be used to create such fusions, for example as described in WO 86/01533 and EP0392745. In another example the effector molecule can increase half life *in vivo,* and/or enhance the delivery of a polypeptide across an epithelial barrier to the immune system. Examples of suitable effector molecules of this type include polymers, albumin, albumin binding proteins or albumin binding compounds such as those described in WO 2005/117984.

In certain aspects, a polypeptide is conjugated to a small molecule toxin. In certain exemplary embodiments, a polypeptide of the disclosure is conjugated to a dolastatin or a dolostatin peptidic analogs or derivatives, *e.g.,* an auristatin (U.S. Pat. Nos. 5,635,483 and 5,780,588). The dolastatin or auristatin drug moiety may be attached to the polypeptide through its N (amino) terminus, C (carboxyl) terminus or internally (WO 02/088172). Exemplary auristatin embodiments include the N-terminus linked monomethylauristatin drug moieties DE and DF, as disclosed in U.S. Patent No. 7,498,298 (disclosing, *e.g.,* linkers and methods of preparing monomethylvaline compounds such as MMAE and MMAF conjugated to linkers).

In other exemplary embodiments, small molecule toxins include but are not limited to calicheamicin, maytansine (U.S. Pat. No. 5,208,020), trichothene, and CC1065. In one embodiment of the disclosure, the polypeptide is conjugated to one or more maytansine molecules (*e.g.,* about 1 to about 10 maytansine molecules per polypeptide molecule). Maytansine may, for example, be converted to May-SS-Me which may be reduced to May-SH3 and reacted with an polypeptide (Chari et al., 1992, Cancer Research 52: 127-131) to generate a maytansinoid- polypeptide or maytansinoid-Fc fusion conjugate. Structural analogues of calicheamicin that can also be used include but are not limited to γ₁¹, γ₃¹, γ₃¹ N-acetyl- γ₁¹, PSAG, and θ₁¹, (Hinman et al., 1993, Cancer Research 53:3336-3342; Lode et al., 1998, Cancer Research 58:2925-2928; U.S. Patent No. 5,714,586; U.S. Patent No. 5,712,374; U.S. Patent No. 5,264,586; U.S. Patent No. 5,773,001).

Polypeptides of the disclosure can also be conjugated to liposomes for targeted delivery (See, *e.g.,* Park et al., 1997, Adv. Pharmacol. 40:399-435; Marty & Schwendener, 2004, Methods in Molecular Medicine 109:389-401).

In one example polypeptides of the present disclosure can be attached to poly(ethyleneglycol) (PEG) moieties. In one particular example the polypeptide is an antibody fragment and the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. See, for example, U.S. Patent No. 5,219,996. Multiple sites can be used to attach two or more PEG molecules. PEG moieties can be covalently linked through a thiol group of at least one cysteine residue located in the antibody fragment. Where a thiol group is used as the point of attachment, appropriately activated effector moieties (for example, thiol selective derivatives such as maleimides and cysteine derivatives) can be used.

The word "label" when used herein refers to a detectable compound or composition which can be conjugated directly or indirectly to a polypeptide of the disclosure. The label can itself be detectable (*e.g.,* radioisotope labels or fluorescent labels) or, in the case of an enzymatic label, can catalyze chemical alteration of a substrate compound or composition which is detectable. Useful fluorescent moieties include, but are not limited to, fluorescein, fluorescein isothiocyanate, rhodamine, 5- dimethylamine-1-napthalenesulfonyl chloride, phycoerythrin and the like. Useful enzymatic labels include, but are not limited to, alkaline phosphatase, horseradish peroxidase, glucose oxidase and the like.

### 6.7. Pharmaceutical Compositions and Therapeutic Methods

Due to their low ADCC activity, polypeptides of the disclosure are particularly useful in the context of immune diseases and disorders, including autoimmune diseases, where cell killing may not be desirable. Examples of such diseases and disorders include Addison's disease, autoimmune diseases of the ear, autoimmune diseases of the eye such as uveitis, autoimmune hepatitis, Crohn's disease, diabetes (Type I), epididymitis, glomerulonephritis, Graves' disease, Guillain-Barre syndrome, Hashimoto's disease, hemolytic anemia, systemic lupus erythematosus (SLE), multiple sclerosis, myasthenia gravis, pemphigus vulgaris, psoriasis, rheumatoid arthritis, sarcoidosis, scleroderma, psoriasis, Sjogren's syndrome, spondyloarthropathies, thyroiditis, ulcerative colitis and/or vasculitis. However, the polypeptides of the disclosure can also be used to treat indications where cell killing is desirable, *e.g.,* oncology indications, particularly when the polypeptide is capable of signaling through the target molecule and/or when conjugated to an effector moiety. The specific indication or indications that are suitable for treatment using an Fc variant polypeptide will depend on the sequence and/or properties of the non-Fc or portion of the Fc variant polypeptide, and can be readily determined by a person of ordinary skill in the art.

In one aspect of the disclosure a variant polypeptide of the disclosure may be an anti-CD40 antibody and is used to treat a CD40-expressing cancer, such as chronic lymphocytic leukemia, Burkitt's lymphoma, multiple myeloma, a T cell lymphoma, Non-Hodgkin's Lymphoma, Hodgkin's Disease, Waldenstrom's macroglobulinemia, Kaposi's sarcoma, ovarian cancer, breast cancer, lung cancer, melanoma, pancreatic cancer, or renal cancer. The anti-CD40 antibody can be a multi-specific antibody.

In another aspect of the disclosure a variant polypeptide of the disclosure may be an anti-CD20 antibody and is used to treat rheumatoid arthritis or multiple sclerosis.

In another aspect of the disclosure a variant polypeptide of the disclosure may be an anti-CD25 antibody and is used to treat multiple sclerosis, psoriasis, asthma, uveitis, ocular inflammation or human T cell leukemia virus-1 associated T-cell leukemia or to prevent organ transplant rejection.

In another aspect of the disclosure a variant polypeptide of the disclosure may be an anti-TNFα antibody and is used to treat rheumatoid arthritis, juvenile idiopathic arthritis, psoriatic arthritis, ankylosing spondylitis, Crohn's disease, ulcerative colitis or plaque psoriasis.

In another aspect of the disclosure a variant polypeptide of the disclosure may be an anti-IL-6 receptor antibody and is used to treat rheumatoid arthritis or Castleman's Disease.

In another aspect of the disclosure a variant polypeptide of the disclosure may be an anti-α4-integrin antibody and is used to treat multiple sclerosis.

In another aspect of the disclosure a variant polypeptide of the disclosure may be an anti-IL-1 antibody and is used to treat Cryopyrin-Associated Periodic Syndromes ("CAPS").

In another aspect of the disclosure a variant polypeptide of the disclosure may be an anti-BAFF antibody and is used to treat systemic lupus erythmatosis or allergy.

The disclosure provides methods of treating any of the foregoing diseases in a patient in need thereof, comprising: administering to the patient an appropriate polypeptide of the disclosure in a therapeutically effective dose.

As used herein, a "therapeutically effective" amount of a polypeptide can be administered as a single dose or over the course of a therapeutic regimen, *e.g.,* over the course of a week, two weeks, three weeks, one month, three months, six months, one year, or longer.

The dosage of a polypeptides of the disclosure to be administered of will vary according to the particular antigen specificity, the type of autoimmune or inflammatory disease, the subject, and the nature and severity of the disease, the physical condition of the subject, the therapeutic regimen (*e.g.,* whether a combination therapeutic agent is used), and the selected route of administration; the appropriate dosage can be readily determined by a person skilled in the art.

For the treatment and/or prophylaxis of autoimmune or inflammatory disease in humans and animals, pharmaceutical compositions comprising polypeptides can be administered to patients (*e.g.,* human subjects) at therapeutically or prophylactically effective dosages (*e.g.,* dosages which result in inhibition of an autoimmune or inflammatory disease and/or relief of autoimmune or inflammatory disease symptoms) using any suitable route of administration, such as injection and other routes of administration known in the art for antibody-based clinical products.

It will be recognized by one of skill in the art that the optimal quantity and spacing of individual dosages of a polypeptide of the disclosure will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop the amount and/or frequency of the dosage can be altered or reduced, in accordance with normal clinical practice.

According to the present disclosure, treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom the polypeptide of the disclosure is administered is preferably a mammal such as a non-primate (*e.g.,* cow, pig, horse, cat, dog, rat, *etc*.) or a primate (*e.g.,* monkey or human). In certain aspects of the disclosure the subject or patient may be a human. In certain aspects, the human is a pediatric patient. In other aspects, the human is an adult patient.

Compositions comprising a polypeptide of the disclosure are provided herein. The compositions will typically be supplied as part of a sterile, pharmaceutical composition that will normally include a pharmaceutically acceptable carrier. This composition can be in any suitable form (depending upon the desired method of administering it to a patient).

Pharmaceutical compositions can be conveniently presented in unit dose forms containing a predetermined amount of a polypeptide of the disclosure per dose. Such a unit can contain for example but without limitation 5 mg to 5 g, for example 10 mg to 1 g, or 20 to 50 mg, 40 mg to 100 mg, or 50 mg to 300 mg. Pharmaceutically acceptable carriers for use in the disclosure can take a wide variety of forms depending, *e.g.,* on the condition to be treated or route of administration.

Therapeutic formulations of the polypeptides of the disclosure can be prepared for storage as lyophilized formulations or aqueous solutions by mixing the polypeptide having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e.g.,* monosodium citrate-disodium citrate mixture, citric acid-trisodium citrate mixture, citric acid-monosodium citrate mixture, *etc*.), succinate buffers (*e.g.,* succinic acid-monosodium succinate mixture, succinic acid-sodium hydroxide mixture, succinic acid-disodium succinate mixture, *etc*.), tartrate buffers (*e.g.,* tartaric acid-sodium tartrate mixture, tartaric acid-potassium tartrate mixture, tartaric acid-sodium hydroxide mixture, *etc*.), fumarate buffers (*e.g.,* fumaric acid-monosodium fumarate mixture, fumaric acid-disodium fumarate mixture, monosodium fumarate-disodium fumarate mixture, *etc*.), gluconate buffers (*e.g.,* gluconic acid-sodium glyconate mixture, gluconic acid-sodium hydroxide mixture, gluconic acid-potassium glyuconate mixture, *etc*.), oxalate buffer (*e.g.,* oxalic acid-sodium oxalate mixture, oxalic acid-sodium hydroxide mixture, oxalic acid-potassium oxalate mixture, *etc*.), lactate buffers (*e.g.,* lactic acid-sodium lactate mixture, lactic acid-sodium hydroxide mixture, lactic acid-potassium lactate mixture, *etc*.) and acetate buffers (*e.g.,* acetic acid-sodium acetate mixture, acetic acid-sodium hydroxide mixture, *etc*.). Additionally, phosphate buffers, histidine buffers and trimethylamine salts such as Tris can be used.

Preservatives can be added to retard microbial growth, and can be added in amounts ranging from 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g.,* chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc.,* organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g.,* peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, *etc*.), polyoxamers (184, 188 *etc*.), Pluronic polyols, polyoxyethylene sorbitan monoethers (TWEEN®-20, TWEEN®-80, *etc*.). Nonionic surfactants can be present in a range of about 0.05 mg/mL to about 1.0 mg/mL, for example about 0.07 mg/mL to about 0.2 mg/mL.

Additional miscellaneous excipients include bulking agents (*e.g.,* starch), chelating agents (*e.g.,* EDTA), antioxidants (*e.g.,* ascorbic acid, methionine, vitamin E), and cosolvents. Further formulations suitable for the polypeptides of the disclosure are disclosed in U.S. Pat. App. No. 2004/0033228 A1.

### 7. EXAMPLES

### Example 1: Construction of a CH2 Point-by-point (PxP) library

Hu1D10, a monoclonal antibody specific for the beta-chain of HLA-DR (Shi et al., 2002, Leuk Lymphoma. 43(6):1303-12) was used as a model system. Synthetic VL and VH domains for Hu1D10 were constructed by a commercial gene synthesis supplier (DNA 2.0 Inc., Menlo Park, CA) and cloned into vector pYA206 to create the pYA206-Hu1D10 plasmid. The vector pYA206 is an Epstein-Barr virus derived episomal vector designed for expression and display of antibodies on the surface of mammalian cells.

84 amino acid positions in the constant region heavy chain domain 2 (CH2) were targeted for mutagenesis using an NNK randomization approach. The NNK coding scheme was used (in which N = A, C, G, or T and K = G or T) because 1) only 32 codons are required to encode all 20 naturally occurring amino acids, 2) only a single stop codon (TAG) is included in the 32, and 3) the maximum degeneracy (number of different codons encoding a single amino acid) is 3, rather than the maximum 6-fold degeneracy that occurs in the complete 64 codon genetic code.

84 different DNA fragments encoding the human Fcγ isotype (Fcγ), each with NNK degeneracy at a different CH2 position, were synthesized by a commercial supplier of synthetic genes (DNA 2.0, Menlo Park, CA). The synthetic Fcγ genes were digested with SalI and NotI restriction enzymes and subcloned into plasmid pYA206-hu1D10. Ligations were transformed into E. coli Top10 cells (Invitrogen, CA) such that at least 10 times more E. coli transformants were obtained than the total number of possible codons in that sub-library. The resulting CH2 library was composed of 1680 total different codons at 84 different positions.

### Example 2: FcγRIIB binding determination

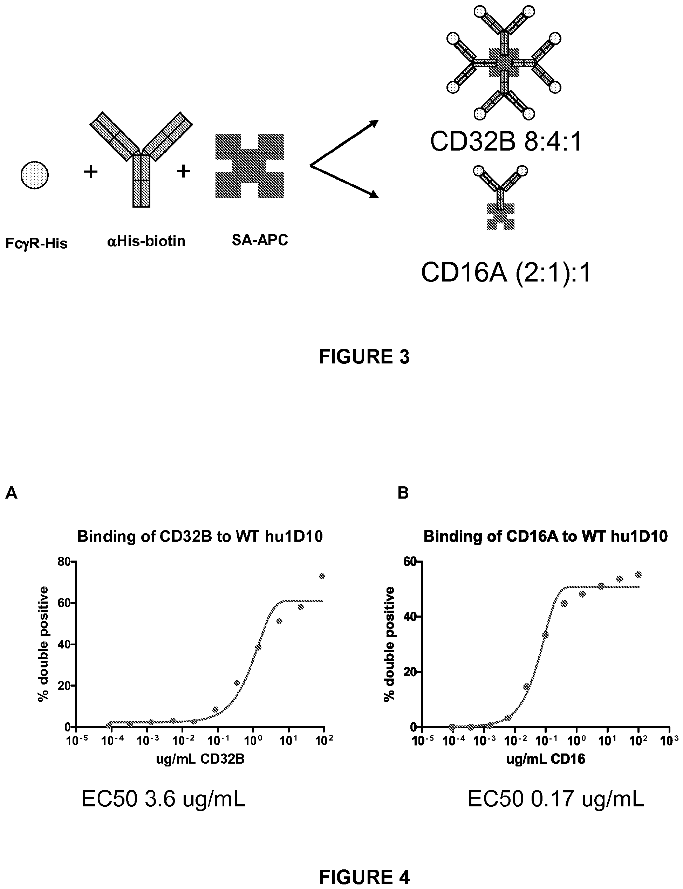

A Fluorescence Activated Cell Sorter (FACS) titration was performed to assess binding of FcγRIIB to unmodified control Fcγ. Because of the low-affinity interaction between FcγRIIB and Fcγ, an 8:4:1 complex was used for flow cytometry (FIGURE 3): Five micrograms of FcγRIIB (R&D Systems, 1875-CD) was pre-incubated with 12.5 µg of biotinylated anti-poly histidine (R&D Systems, BAM050), then added to 3.5 µg of Streptavidin-APC (Southern Biotech, cat # 7100-11L). This complex was then serially diluted 4-fold and combined with goat anti-human kappa-PE (Southern Biotech, 2060-09). To determine the EC₅₀ of FcγR-complex binding, 100 µl of complex was added to 2 x 10⁵ cells at each concentration and incubated for 1hr. After three washes in a FACS buffer, cells were analyzed by flow cytometry in a FACSCalibur (BD Biosciences). The percent of cells in the double positive quadrant was determined and plotted against FcγRIIB concentration. The EC₅₀ was determined to be 3.6 µg/mL (FIGURE 4A).

### Example 3: FcγRIIIA binding determination

A FACS titration was performed to assess binding of FcγRIIIA to Fcγ. Because of the low-affinity interaction between FcγRIIIA and Fcγ, a 2:1:1 complex was used for flow cytometry (FIGURE 3). 9 µg of FcγRIIIA (R&D Systems, cat # 4325-FC) was pre-incubated with 15 µg of biotinylated anti-poly histidine (R&D Systems, BAM050), then added to 15 µg of Streptavidin-APC (Southern Biotech, cat # 7100-11L). This complex was then serially diluted 4-fold and combined with goat anti-human kappa-PE (Southern Biotech, 2060-09). To determine the EC₅₀ of FcyR-complex binding, 100 µl of complex was added to 2 x 10⁵ cells at each concentration and incubated for 1hr. After three washes in a FACS buffer, cells were analyzed by flow cytometry in a FACSCalibur (BD Bioscience). The percent of cells in the double positive quadrant was determined and plotted against FcγRIIIA concentration. The EC₅₀ was determined to be 0.17 ug/mL (Fig 2B).

### Example 4: Fluorescence activated cell sorting (FACS) of the CH2 library

The CH2 library was transfected into 293c18 cells with 0.5 µg library plasmid, 100 µg pACYC184 carrier plasmid and 250 µl lipofectamine; selected using 0.8 µg/ml puromycin after 2 days, and cultured for an additional 18 days prior to FACS sorting.

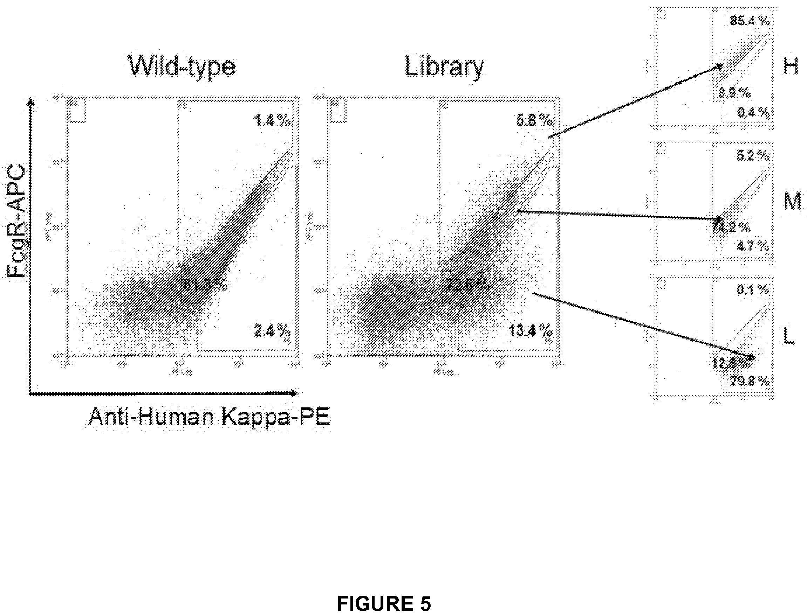

Cells were co-stained with 1:200 PE-labeled anti-human Kappa-PE antibody (Southern Biotech) and either FcγRIIIA- or FcγRIIB-complex (FIGURE 3) at or below 50% maximal binding as determined by titration. A minimum of 1 x 10⁵ cells were sorted from three populations - high (H), medium (M), and low (L) - based on differential binding to the FcγR. A representative FACS sort result is shown in FIGURE 5. Variants with desired properties were enriched in the H-gate in the FcγRIIB binding and enriched in L-gate in the FcγRIIIA binding.

### Example 5: Massively parallel sequencing of the "expressed" and "sorted" populations

Plasmids were recovered from the sorted H, M, and L cell populations as described in Example 4, and PCR amplification was performed to prepare short amplicons suitable for massively parallel sequencing. Amplicons were then sequenced using the Genome Sequencer FLX as directed by manufacturer (454 Life Sciences, Branford, CT). Approximately 800,000 individual sequences were determined for each population of the FACS-sorted cells.

The sequences were examined and the number of times each point mutation was found in the "expressed" and "sorted" population was tabulated. Each amino acid codon was initially identified and tabulated. For amino acids with more than one codon, the occurrence of the different codons for each amino acid were added together to make an overall summary of the behavior of that amino acid variant in each subpopulation. For the 3-way sort of the CH2 library to assess binding to FcγRIIIA or FcγRIIB, an Enrichment Ratio (ER) score was assigned for each codon variant. The ER denotes how much more or less frequent the variant is found in the H population compared to its overall frequency. Similarly, Enrichment Ratios can be calculated for each variant in each of the M, and L populations. Higher affinity variants are expected to be enriched in the H population (ER>1) and depleted (ER<1) in the L population. Conversely, lower affinity mutants are expected to be depleted in the H population (ER<1) and enriched in the L population (ER>1). It is possible to identify higher, lower, and neutral affinity variants simply by observing the Enrichment Ratios for the H population.

### Example 6: Identification of Point Mutants With Desired Properties

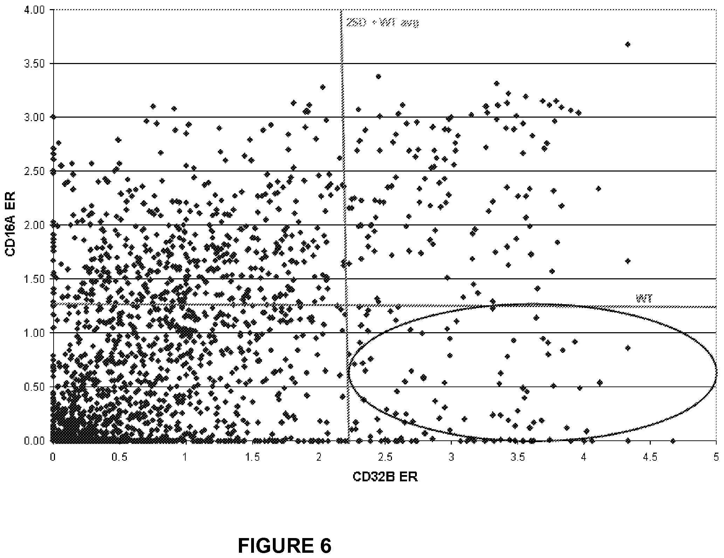

Comprehensive mutagenesis of 84 positions in CH2 identified variants with a significantly increased FcγRIIB binding and decreased binding to FcγRIIIA (lower than WT), lower right quadrant in FIGURE 6. In this example, 2 standard deviations above the wild-type average was taken to be significantly increased, and lower than the wild-type average was taken to be decreased.

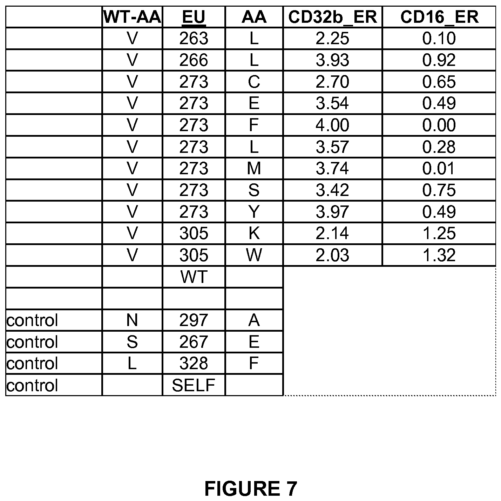

Positions and substitutions that were identified as having significantly increased FcγRIIB binding and decreased binding to FcγRIIIA are shown in FIGURE 7. Variant human IgG were expressed with the Hu1D10 binding domain either as soluble IgG1 or surface-expressed on 293c18 cells.

### Example 7: Confirmation of improved binding using single-point (one-point) FACS

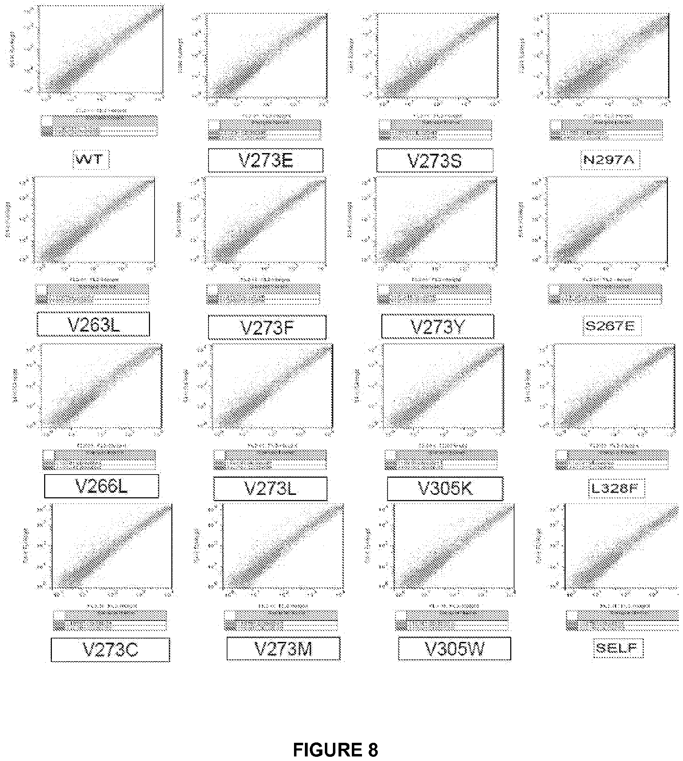

To confirm improved binding to FcγRIIB, one-point FACS analysis was performed comparing variants to parent IgG expressed on 293c18 cells. IgG variant-expressing 293c18 and controls were stained with FcγRIIB complex at the EC50 concentration. 293c18 expressing an Fc variant containing the N297A modification was used as a negative control. S267E, L328F, and the double mutant "SELF" were included as positive controls. Samples were analyzed by flow cytometry in a FACSCalibur device and the result for each variant was plotted against the wild-type, with FcγR binding on the y-axis and IgG expression on the x-axis (FIGURE 8).

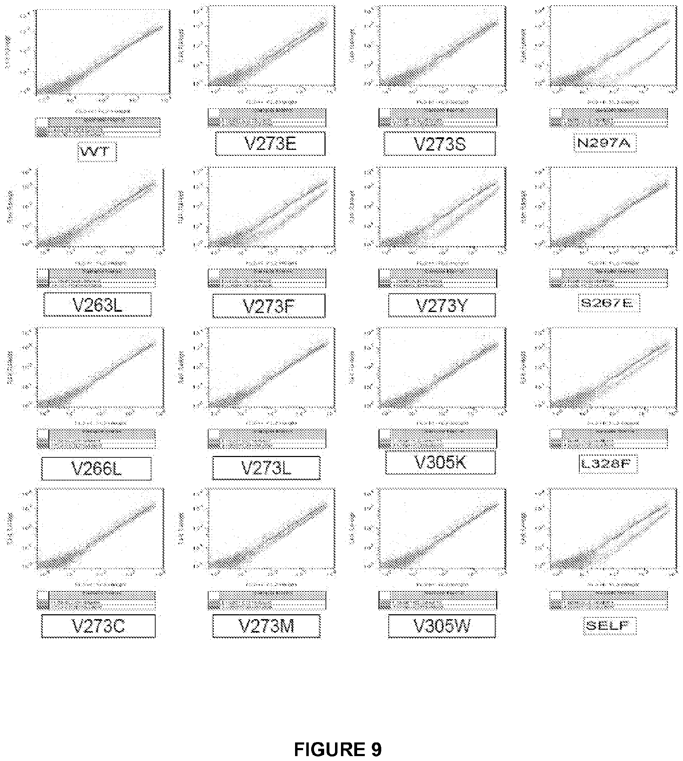

To confirm decreased binding to FcγRIIIA, one-point FACS analysis was performed comparing variants to parent IgG expressed on 293c18 cells. IgG variant-expressing 293c18 and controls were stained with FcγRIIIA complex at the EC50 concentration. 293c18 expressing N297A, S267E, L328F, and the double mutant "SELF" were used as controls. Samples were analyzed by flow cytometry using a FACSCalibur device and the result for each variant was plotted against the WT, with FcγR binding on the y-axis and IgG expression on the x-axis (FIGURE 9). The variants with the most significant downward shift were found to be V263L, V273E, V273F, V273M, V273S, and V273Y.

### Example 8: Binding of variants to FcγR-expressing cells

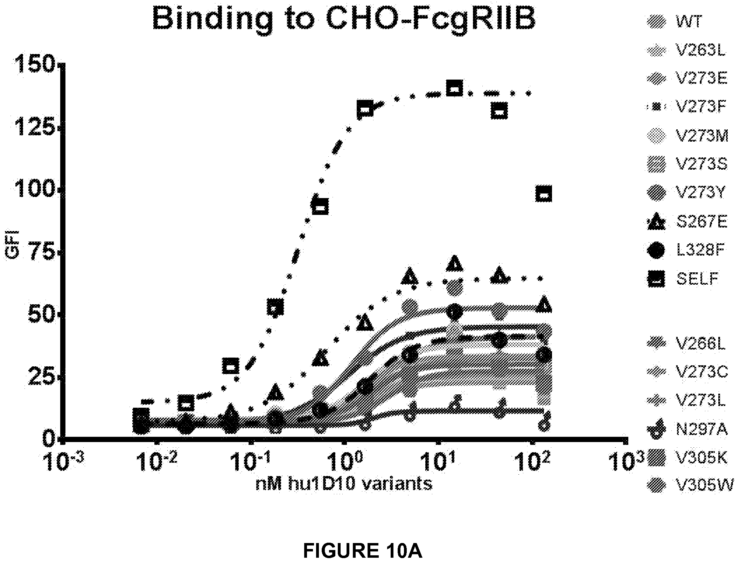

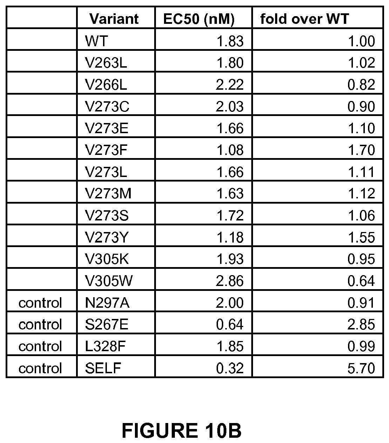

Hu1D10 IgG variant antibodies were expressed in soluble form, purified, and then used to assess binding to CHO cells expressing FcγRIIB. IgG variants were serially-diluted 3-fold starting at 20 µg/mL, or 133 nM, then added to 2x10⁵ cells/test. Anti-human kappa antibody was used to detect variant IgG binding. Samples were analyzed in a FACSCalibur and fluorescence was plotted against IgG concentration. FIGURE 10 confirms that all the variants have a higher maximal binding to FcγRIIB than the wild-type antibody.

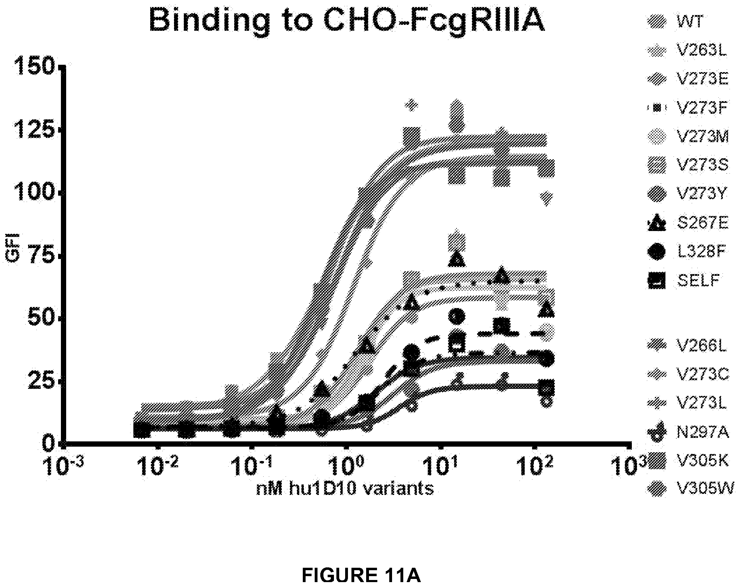

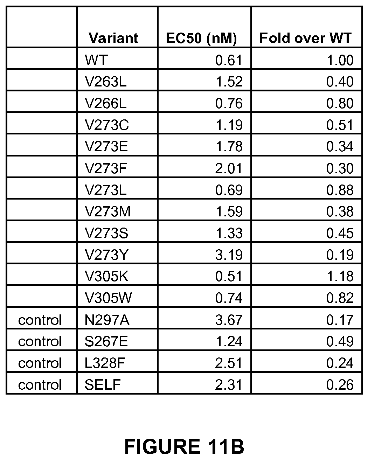

Hu1D10 IgG variants were purified and used to assess binding to FcγRIIIA CHO transfectants. IgG variants were serially-diluted 3-fold starting at 20ug/mL, or 133nM and then added to 2x10⁵ cells/test. A secondary stain of anti-human kappa antibody was used to detect variant IgG binding. Samples were analyzed in a FACSCalibur and fluorescence was plotted against IgG concentration in FIGURE 11. All variants bound equivalently or less well than wild-type Fc-containing antibody to FcγRIIIA.

### Example 9: FACS-based antibody-dependent cell-mediated cytotoxicity

A non-radioactive antibody dependent cell cytotoxicity (ADCC) assay was optimized and used to test Hu1D10 IgG variants. Raji cells, and PBMC purified from freshly-drawn whole blood were used as target and effector cells, respectively, at a 1:40 ratio.

The Raji cells were washed and resuspended at 10⁶ cells/mL in PBS, then incubated with a 1:2000 dilution of CSFE (Cell Technology, Inc., part 4002) for 30 minutes. CFSE-loaded Raji cells were then washed and resuspended to 4x10⁵/mL in growth medium consisting of RPMI + 10% heat-inactivated FBS. 50 µL of cell suspension was added to each well of a V-bottom plate. 50 µL of three-fold serially diluted IgG variants was added to each well, starting at 18 µg/mL.

PBMCs were purified from freshly-drawn heparinized blood centrifuged over Ficoll-Paque (GE, 17-1440-02) at 665 RCF for 30 minutes. The PBMC layer was collected and washed three times in PBS + 10% FBS, first wash at 1350 RCF for 15 minutes, second wash at 225 RCF for 10 minutes, and the third wash at 225 RCF for 10 minutes. After the final wash, cells were resuspended in growth media and counted using a Vi-Cell Rx. Cells were centrifuged and resuspended to 8x10⁶ cells/mL in growth media. 100 µL of cell suspension was added to each well of the target/IgG suspension and incubated at 37C for four hours. Cell suspensions were stained with 1:5 dilution of 7AAD (BD Biosciences, catalog number 559925) and incubated for 30 minutes. To determine spontaneous death of target cells, CSFE-loaded Raji cells were incubated with media only (0 mg/mL IgG, no PBMC), then stained with 7AAD. Samples were analyzed in a FACSCalibur.

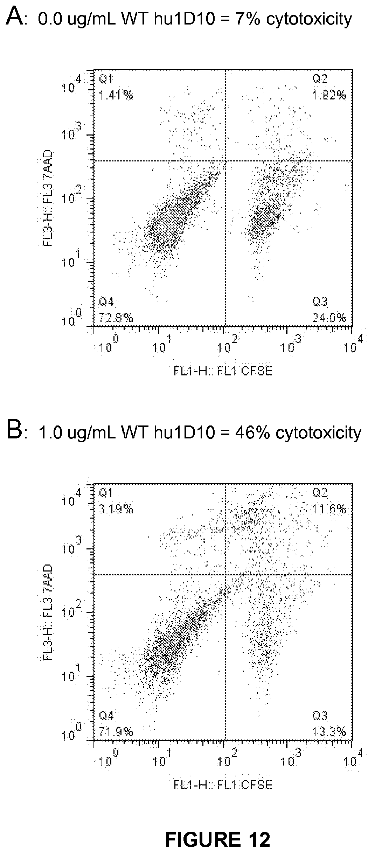

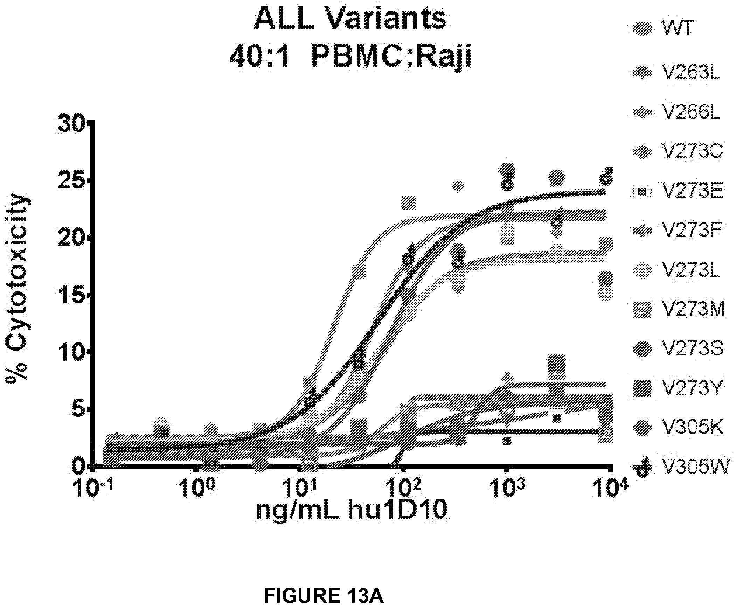

FACS data were graphed for each sample with CFSE (FL1) on the x-axis and 7AAD (FL3) in the y-axis. A quadrant was drawn, discriminating target cells (CFSE+) from PBMC (CFSE-), as well as 7AAD-positive from 7AAD-negative cells (FIGURE 12). The number of cells in the upper right quadrant was defined as "dead" and those in lower right quadrant as "live." Percent cytotoxicity was calculated, subtracting spontaneous death. The percent cytotoxicity was graphed against IgG concentration to determine the EC₅₀ (FIGURE 13A).

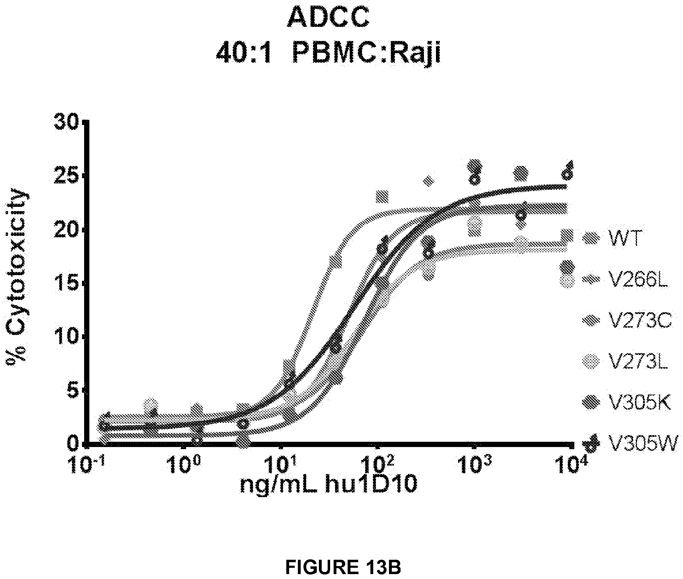

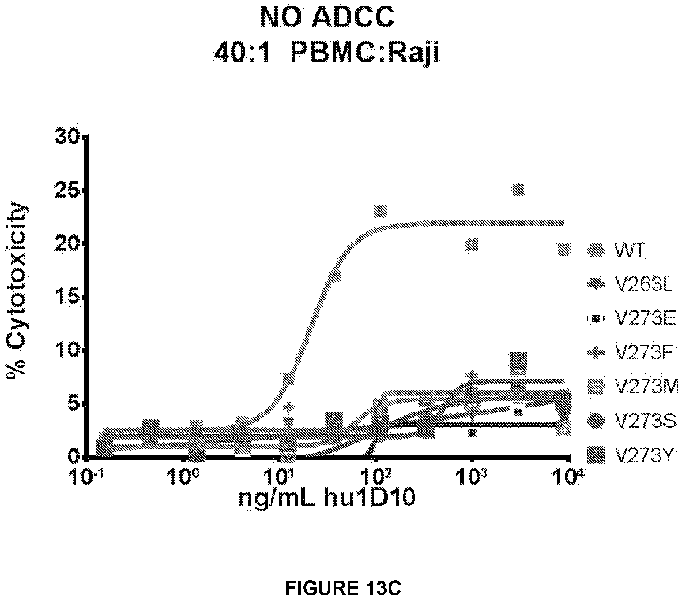

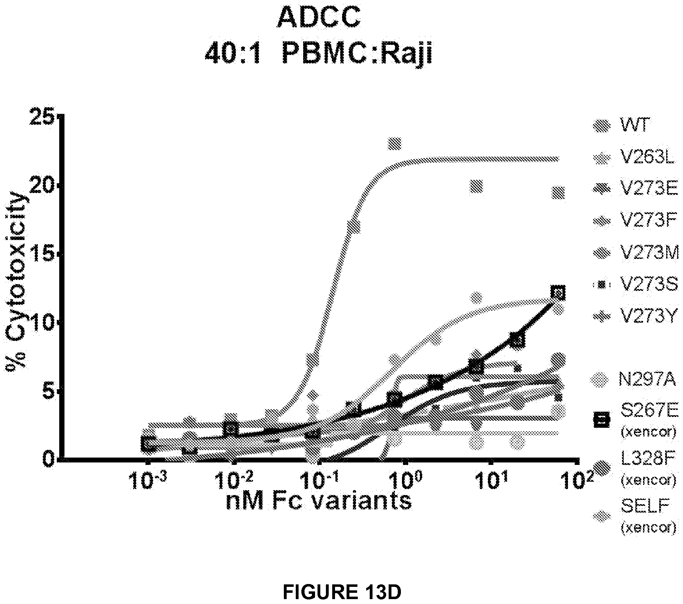

Hu1D10 variants were grouped based on ADCC activity. FIGURE 13B shows FcyRIIB up-mutants having some ADCC activity, though lower than wild-type; FIGURE13C shows variants with little to no ADCC activity. FIGURE13D compares the non-ADCC hu1D10 variants to substitutions that result in decreased binding to FcγRIIIA (S267E, L328F, double mutant "SELF") according to literature. FIGURE14D shows that V263L, V273E, V273F, V273M, V273S, and V273Y elicited comparable responses to L328F and lower ADCC responses than S267E and SELF.

### Example 10: Antibody Dependent Cell-Mediated Cytotoxicity of Fc Binding Variants of an Anti-CD40 mAb

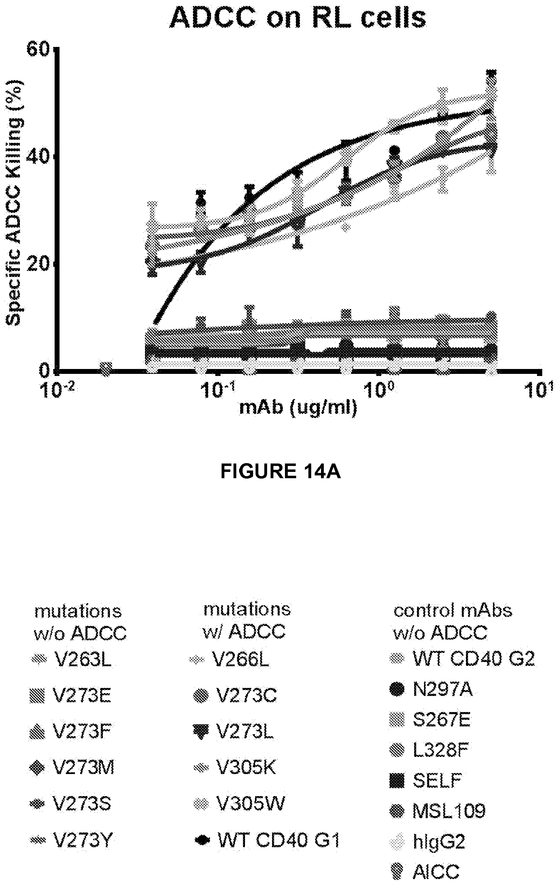

An antibody-dependent cell-mediated cytotoxicity (ADCC) assay was designed according to a standard protocol (Law et al., 2005, Cancer Res. 65:8331-8) using an anti-CD40 monoclonal antibody with modifications in Fcγ. Lymphoma RL cells were labeled with ⁵¹Chromium for 1 hour as target cells. PBMC were used as effector cells and were mixed with target at a 50:1 ratio. Anti-CD40 was diluted in series and applied to target/effector cell mixtures. After 4 hours incubation at 37C, 5% CO₂, 100 µl of culture supernatant was harvested and radioactivity released was monitored by gamma counter. Cultures without antibody were recorded as media treated negative controls, and the maximum ⁵¹Chromium release was achieved by Triton X100 treatment of labeled target cells. The final percent of cytotoxicity was calculated using the formula: ((sample - (target + media))/((target + Triton)- (target + media)))*100. The Fcγ variants V263L, V273E, V273F, V273M, V273S, and V273Y did not induce ADCC activity (FIGURE 14).

### Example 11: Functional Activity of Anti-CD40 mAb with Fcγ Substitutions

To test whether the differential binding to FcγRIIB affects immune activation of dendritic cells, anti-CD40 monoclonal antibodies were constructed with the substitutions in Fcγ and tested in an IL-12p70 secretion assay.

Whole blood from healthy human donors, diluted with an equal volume of PBS, was added to a Leucosep (Greiner Bio One) tube, containing Ficoll-Paque Plus below the frit (15 mL). The blood was then centrifuged at 1,000g for 15 minutes without brake. PBMC were collected and washed once with PBS, centrifuged at 1,300 rpm for 5 minutes at room temperature, and washed once with RPMI 1640. Cells were re-suspended in SN12C culture medium (RPMI1640+10% heat-inactivated FBS).

Generation of monocyte-derived immature dendritic cell (moDC): Monocytes were isolated from PBMC with an enrichment kit from StemCell and were cultured in StemSep serum free medium supplemented with 10 ng/ml GM-CSF and 20 ng/ml IL-4 at 37 C, 5% CO2 for 6 days. Fresh GM-CSF and IL-4 were added to the culture at day 3 to help maintaining DC differentiation. After 6 days culture, monocyte-derived immature DC were subject to FACS analysis to verify immature DC phenotype: Lin-, CD80/CD86+, HLA-DR+, CD11C+.

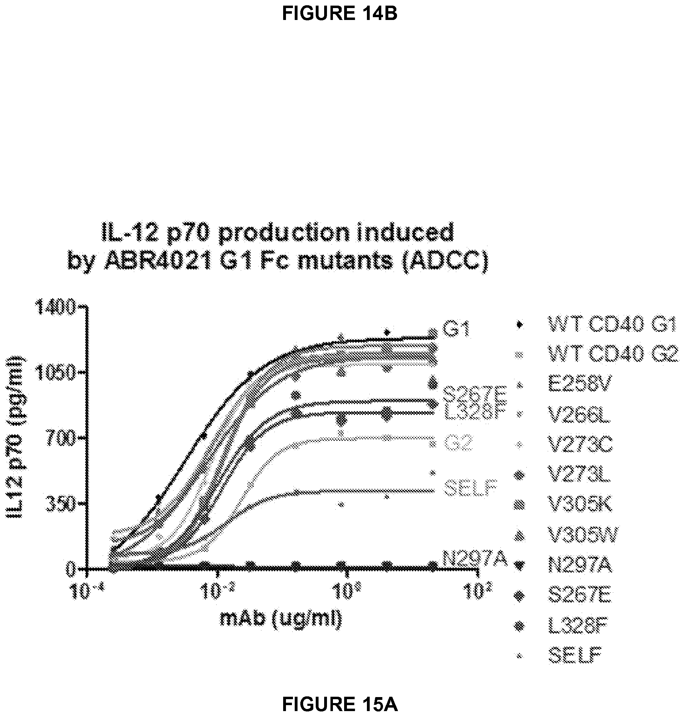

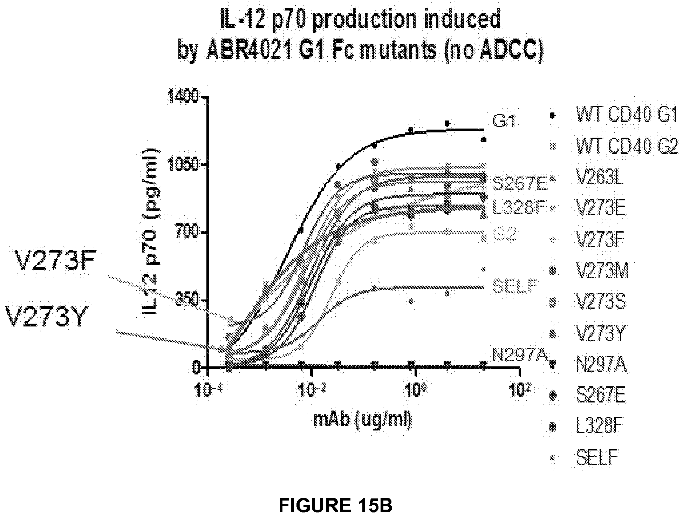

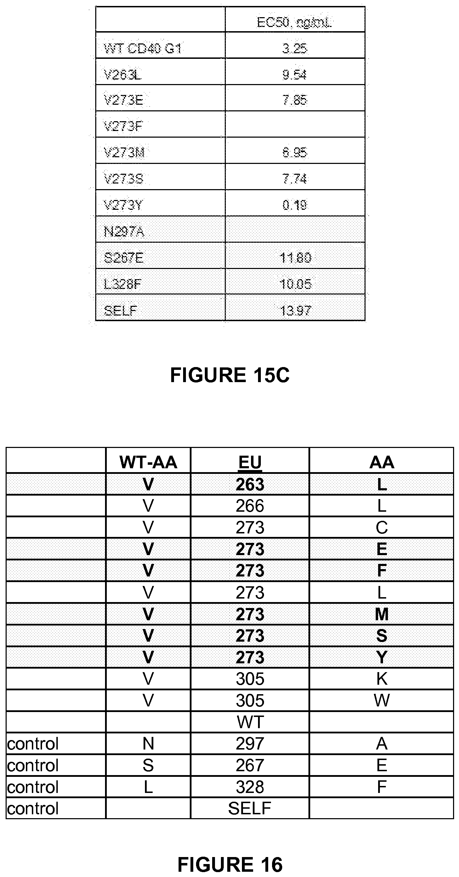

Monitoring agonistic activity of anti-CD40 in stimulating IL-12p70 from moDC: Immature moDC were stimulated with anti-CD40 and primed with IFNγ for 48 hours in StemSep serum free medium supplemented with GM-CSF and IL-4. The culture supernatant was harvest and assayed for IL-12p70 production by a commercially available ELISA kit. FIGURE 15 shows IL-12p70 production of ADCC-inducing (FIGURE 15A) and non-ADCC-inducing (FIGURE 15B) variants. Of the non-ADCC variants, V273F and V273Y showed the most enhanced dendritic cells activation as measured by IL-12p70 secretion (FIGURE 15C).

## Claims

1. An antibody comprising a CH2 domain of SEQ ID NO:2, which has relative to the CH2 domain of SEQ ID NO:2
(a) a substitution or set of substitutions according to the EU index as in Kabat selected from:
(i) V273E,
(ii) V273F,
(iii) V273M,
(iv) V273S,
(v) V273Y,
(vi) V263L and V273E,
(vii) V263L and V273F,
(viii) V263L and V273M,
(ix) V263L and V273S, and
(x) V263L and V273Y;
(b) the substitution V263L according to the EU index as in Kabat, wherein the antibody binds specifically to CD40; or
(c) the substitution V263L according to the EU index as in Kabat, wherein the antibody binds specifically to a costimulatory molecule selected from CD28, PD-1, CTLA-4, CD80, CD86, TIM3, OX40, 4-1BB, GITR, CD27, B7-H4, and DC-SIGN;
optionally wherein the CH2 domain is part of an Fc domain comprising T250Q and M428L according to the EU index as in Kabat.

2. The antibody of claim 1, wherein the antibody in (a) specifically binds to CD40, CD25, CD3, an HLA molecule, a costimulatory molecule, a cytokine, a chemokine, an adhesion molecule, an activation marker, or an immunomodulatory protein.

3. A conjugate compound comprising the antibody of claim 1 linked to an effector moiety or a detectable label.

4. A pharmaceutical composition comprising the antibody of claim 1 and a pharmaceutically acceptable carrier, or the conjugate compound of claim 3.

5. A vector comprising a nucleic acid comprising a nucleotide sequence encoding the antibody of claim 1.

6. A prokaryotic host cell comprising the vector of claim 5.

7. A method of producing an antibody, comprising: (a) culturing a eukaryotic host cell engineered to express a nucleic acid comprising a nucleotide sequence encoding the antibody of claim 1 and (b) recovering the antibody.

8. An antibody according to claim 1, a conjugate compound according to claim 3, or a pharmaceutical composition according to claim 4, for use in treating an immune disorder or a cancer in a patient in need thereof.

9. The antibody, pharmaceutical composition, or conjugate compound for use according to claim 8, wherein the antibody is an anti-CD40 antibody and wherein the cancer is a hematological cancer, optionally selected from chronic lymphocytic leukemia, Burkitt's lymphoma, multiple myeloma, a T cell lymphoma, Non-Hodgkin's Lymphoma, Hodgkin's Disease, Waldenstrom's macroglobulinemia, Kaposi's sarcoma, or a solid tumor cancer, optionally selected from ovarian cancer, breast cancer, lung cancer, melanoma, pancreatic cancer, and renal cancer.

10. The antibody of claim 2, wherein the costimulatory molecule is CD28, PD-1, CTLA-4, CD80, CD86, TIM3, OX40, 4-1BB, GITR, CD27, B7-H4, or DC-SIGN.

11. The antibody of claim 1, wherein the antibody is a monoclonal antibody.

12. The antibody of claim 1, wherein the antibody is a bispecific antibody.

13. The antibody of claim 11 or 12, wherein the antibody is humanized.

14. The antibody of claim 13, wherein the antibody specifically binds to CD40.

15. The antibody of claim 14, wherein the antibody has relative to the CH2 domain of SEQ ID NO: 2 the substitution V273E.

## Patentansprüche

1. Antikörper, umfassend eine CH2-Domäne von SEQ ID NO:2, die relativ zur CH2-Domäne von SEQ ID NO:2 aufweist
(a) eine Substitution oder eine Reihe von Substitutionen gemäß EU-Index wie in Kabat, die ausgewählt sind aus:
(i) V273E,
(ii) V273F,
(iii) V273M,
(iv) V273S,
(v) V273Y,
(vi) V263L und V273E,
(vii) V263L und V273F,
(viii) V263L und V273M,
(ix) V263L und V273S und
(x) V263L und V273Y;
(b) die Substitution V263L gemäß EU-Index wie in Kabat, wobei der Antikörper speziell an CD40 bindet; oder
(c) die Substitution V263L gemäß dem EU-Index wie in Kabat, wobei der Antikörper speziell an ein kostimulatorisches Molekül bindet, das ausgewählt ist aus CD28, PD-1, CTLA-4, CD80, CD86, TIM3, OX40, 4-1BB, GITR, CD27, B7-H4 und DC-SIGN;
wobei optional die CH2-Domäne Teil einer Fc-Domäne ist, die gemäß EU-Index wie in Kabat T250Q und M428L umfasst.

2. Antikörper nach Anspruch 1, wobei der Antikörper in (a) speziell an CD40, CD25, CD3, ein HLA-Molekül, ein kostimulatorisches Molekül, ein Zytokin, ein Chemokin, ein Adhäsionsmolekül, einen Aktivierungsmarker oder ein immunmodulatorisches Protein bindet.

3. Konjugatverbindung, die den Antikörper nach Anspruch 1 umfasst, der mit einer funktionellen Effektorgruppe oder einer nachweisbaren Markierung verbunden ist.

4. Pharmazeutische Zusammensetzung, die den Antikörper nach Anspruch 1 und einen pharmazeutisch verträglichen Träger oder die Konjugatverbindung nach Anspruch 3 umfasst.

5. Vektor, der eine Nukleinsäure umfasst, die eine den Antikörper nach Anspruch 1 codierende Nukleotidsequenz umfasst.

6. Prokaryotische Wirtszelle, die den Vektor nach Anspruch 5 umfasst.

7. Verfahren zum Herstellen eines Antikörpers, umfassend: (a) Züchten einer eukaryotischen Wirtszelle, die ausgelegt ist, eine Nukleinsäure zu exprimieren, welche eine Nukleotidsequenz umfasst, die den Antikörper nach Anspruch 1 codiert, und (b) Wiedergewinnen des Antikörpers.

8. Antikörper nach Anspruch 1, eine Konjugatverbindung nach Anspruch 3 oder eine pharmazeutische Zusammensetzung nach Anspruch 4 zur Verwendung bei der Behandlung einer Immunkrankheit oder von Krebs bei einem betroffenen Patienten.

9. Antikörper, pharmazeutische Zusammensetzung oder Konjugatverbindung zur Verwendung nach Anspruch 8, wobei der Antikörper ein anti-CD40-Antikörper ist, und wobei der Krebs ein hämatologischer Krebs ist, welcher optional ausgewählt ist aus chronischer lymphatischer Leukämie, Burkitt-Lymphom, multiplem Myelom, einem T-Zellen-Lymphom, Non-Hodgkin-Lymphom, Morbus Hodgkin, Waldenström-Krankheit, Kaposisarkom oder einem soliden Tumor, der optional ausgewählt ist aus Ovarialkrebs, Brustkrebs, Lungenkrebs, Melanom, Bauchspeicheldrüsenkrebs und Nierenkrebs.

10. Antikörper nach Anspruch 2, wobei das kostimulatorische Molekül CD28, PD-1, CTLA-4, CD80, CD86, TIM3, OX40, 4-1BB, GITR, CD27, B7-H4, oder DC-SIGN ist.

11. Antikörper nach Anspruch 1, wobei der Antikörper ein monoklonaler Antikörper ist.

12. Antikörper nach Anspruch 1, wobei der Antikörper ein bispezifischer Antikörper ist.

13. Antikörper nach Anspruch 11 oder 12, wobei der Antikörper humanisiert ist.

14. Antikörper nach Anspruch 13, wobei der Antikörper speziell an CD40 bindet.

15. Antikörper nach Anspruch 14, wobei der Antikörper relativ zur CH2-Domäne von SEQ ID NO: 2 die Substitution V273E aufweist.

## Revendications

1. Anticorps comprenant un domaine CH2 de SEQ ID NO : 2, qui a, par rapport au domaine CH2 de SEQ ID NO : 2
(a) une substitution ou un ensemble de substitutions selon l'indice UE tel que dans Kabat choisies parmi :
(i) V273E,
(ii) V273F,
(iii) V273M,
(iv) V273S,
(v) V273Y,
(vi) V263L et V273E,
(vii) V263L et V273F,
(viii) V263L et V273M,
(ix) V263L et V273S, et
(x) V263L et V273Y ;
(b) la substitution V263L selon l'indice UE tel que dans Kabat, l'anticorps se liant spécifiquement à CD40 ; ou
(c) la substitution V263L selon l'indice UE tel que dans Kabat, l'anticorps se liant spécifiquement à une molécule de co-stimulation choisie parmi CD28, PD-1, CTLA-4, CD80, CD86, TIM3, 0X40, 4-1BB, GITR, CD27, B7-H4 et DC- SIGN ;
le domaine CH2 faisant éventuellement partie d'un domaine Fc comprenant T250Q et M428L selon l'indice UE tel que dans Kabat.

2. Anticorps de la revendication 1, l'anticorps dans (a) se liant spécifiquement à CD40, CD25, CD3 une molécule de HLA, une molécule de co-stimulation, une cytokine, une chimiokine, une molécule d'adhésion, un marqueur d'activation ou une protéine d'immunomodulation.

3. Composé conjugué comprenant l'anticorps de la revendication 1 lié à une fraction d'effecteur ou un marqueur détectable.

4. Composition pharmaceutique comprenant l'anticorps de la revendication 1 et un support pharmaceutiquement acceptable, ou le composé conjugué de la revendication 3.

5. Vecteur comprenant un acide nucléique comprenant une séquence nucléotidique codant pour l'anticorps de la revendication 1.

6. Celle hôte procaryote comprenant le vecteur de la revendication 5.

7. Procédé de production d'un anticorps, comprenant : (a) la mise en culture d'une cellule hôte eucaryote modifiée pour exprimer un acide nucléique comprenant une séquence nucléotidique codant pour l'anticorps de la revendication 1 et (b) le recueil de l'anticorps.

8. Anticorps selon la revendication 1, composé conjugué selon la revendication 3, ou composition pharmaceutique selon la revendication 4, destiné à une utilisation dans le traitement d'un trouble immunitaire ou d'un cancer chez un patient qui le nécessite.

9. Anticorps, composition pharmaceutique ou composé conjugué destiné à une utilisation selon la revendication 8, l'anticorps étant un anticorps anti-CD40 et le cancer étant un cancer hématologique, éventuellement choisi parmi la leucémie lymphoïde chronique, le lymphome de Burkitt, le myélome multiple, le lymphome T, le lymphome non hodgkinien, la maladie de Hodgkin, la macroglobulinémie de Waldenstrôm, le sarcome de Kaposi ou un cancer à tumeur solide, éventuellement choisi parmi le cancer de l'ovaire, le cancer du sein, le cancer du poumon, le mélanome, le cancer du pancréas et le cancer du rein.

10. Anticorps de la revendication 2, dans lequel la molécule de co-stimulation est CD28, PD-1, CTLA-4, CD80, CD86, TIM3, OX40, 4-BB, GITR, CD27, B7-H4 ou DC-SIGN.

11. Anticorps de la revendication 1, l'anticorps étant un anticorps monoclonal.

12. Anticorps de la revendication 1, l'anticorps étant un anticorps bispécifique.

13. Anticorps de la revendication 11 ou 12, l'anticorps étant humanisé.

14. Anticorps de la revendication 13, l'anticorps se liant spécifiquement à CD40.

15. Anticorps de la revendication 14, l'anticorps ayant, par rapport au domaine CH2 de SEQ ID NO : 2 la substitution V273E.


### SEQUENCE LISTING

<110> ABBVIE BIOTHERAPEUTICS INC.
<120> FC VARIANTS
<130> 381493-884WO (125234)
<140>
   <141>
<150> 61/791,624
   <151> 2013-03-15
<160> 38
<170> PatentIn version 3.5
<210> 1
   <211> 227
   <212> PRT
   <213> Homo sapiens
<400> 1
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 2
   <211> 110
   <212> PRT
   <213> Homo sapiens
<400> 2
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 3
   <211> 112
   <212> PRT
   <213> Artificial Sequence
<220>
   <221> source
   <223> /note="Description of Artificial Sequence: Synthetic polypeptide"
<400> 3
   Asp Val Val Val Thr Gln Thr Pro Leu Ser Leu Pro Val Ser Leu Gly
   1               5                   10                  15
   Ala Gln Ala Ser Ile Ser Cys Arg Ser Ser Gln Ser Leu Val His Ser
               20                  25                  30
   Asn Gly Asn Thr Phe Leu His Trp Tyr Leu Gln Lys Pro Gly Gln Ser
           35                  40                  45
   Pro Lys Leu Leu Ile Tyr Thr Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Phe Cys Ser Gln Thr
                   85                  90                  95
   Thr His Val Pro Trp Thr Phe Gly Gly Gly Thr Lys Leu Glu Ile Gln
               100                 105                 110
<210> 4
   <211> 114
   <212> PRT
   <213> Artificial Sequence
<220>
   <221> source
   <223> /note="Description of Artificial Sequence: Synthetic polypeptide"
<400> 4
   Glu Val Gln Leu Gln Gln Ser Gly Pro Asp Leu Val Lys Pro Gly Ala
   1               5                   10                  15
   Ser Val Lys Ile Ser Cys Lys Ala Ser Gly Tyr Ser Phe Thr Gly Tyr
               20                  25                  30
   Tyr Ile His Met Val Lys Gln Ser Lys Gly His Ser Leu Glu Trp Ile
           35                  40                  45
   Gly Arg Val Ile Pro Asn Asn Gly Gly Thr Ser Tyr Asn Gln Lys Phe
       50                  55                  60
   Lys Gly Lys Ala Ile Leu Thr Val Asp Lys Ser Ser Ser Thr Ala Tyr
   65                  70                  75                  80
   Met Glu Leu Arg Ser Leu Thr Ser Glu Asp Ser Ala Val Tyr Tyr Cys
   85 90 95
   Ala Arg Glu Gly Ile Tyr Trp Trp Gly His Gly Thr Thr Leu Thr Val
   100 105 110
   Ser Ser
<210> 5
   <211> 106
   <212> PRT
   <213> Artificial Sequence
<220>
   <221> source
   <223> /note="Description of Artificial Sequence: Synthetic polypeptide"
<400> 5
<210> 6
   <211> 121
   <212> PRT
   <213> Artificial Sequence
<220>
   <221> source
   <223> /note="Description of Artificial Sequence: Synthetic polypeptide"
<400> 6
<210> 7
   <211> 106
   <212> PRT
   <213> Artificial Sequence
<220>
   <221> source
   <223> /note="Description of Artificial Sequence: Synthetic polypeptide"
<400> 7
   Asp Ile Gln Met Thr Gln Ser Pro Ser Thr Leu Ser Ala Ser Val Gly
   1               5                   10                  15
   Asp Arg Val Thr Ile Thr Cys Ser Ala Ser Ser Ser Ile Ser Tyr Met
               20                  25                  30
   His Trp Tyr Gln Gln Lys Pro Gly Lys Ala Pro Lys Leu Leu Ile Tyr
           35                  40                  45
   Thr Thr Ser Asn Leu Ala Ser Gly Val Pro Ala Arg Phe Ser Gly Ser
       50                  55                  60
   Gly Ser Gly Thr Glu Phe Thr Leu Thr Ile Ser Ser Leu Gln Pro Asp
   65                  70                  75                  80
   Asp Phe Ala Thr Tyr Tyr Cys His Gln Arg Ser Thr Tyr Pro Leu Thr
                   85                  90                  95
   Phe Gly Gln Gly Thr Lys Val Glu Val Lys
               100                 105
<210> 8
   <211> 116
   <212> PRT
   <213> Artificial Sequence
<220>
   <221> source
   <223> /note="Description of Artificial Sequence: Synthetic polypeptide"
<400> 8
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Thr Ser Tyr
               20                  25                  30
   Arg Met His Trp Val Arg Gln Ala Pro Gly Gln Gly Leu Glu Trp Ile
           35                  40                  45
   Gly Tyr Ile Asn Pro Ser Thr Gly Tyr Thr Glu Tyr Asn Gln Lys Phe
       50                  55                  60
   Lys Asp Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Gly Val Phe Asp Tyr Trp Gly Gln Gly Thr Leu Val
               100                 105                 110
   Thr Val Ser Ser
           115
<210> 9
   <211> 104
   <212> PRT
   <213> Artificial Sequence
<220>
   <221> source
   <223> /note="Description of Artificial Sequence: Synthetic polypeptide"
<400> 9
   Gln Ile Val Leu Thr Gln Ser Pro Ala Ile Met Ser Ala Ser Pro Gly
   1               5                   10                  15
   Glu Lys Val Thr Met Thr Cys Ser Ala Ser Ser Ser Ile Ser Tyr Met
               20                  25                  30
   Gln Trp Tyr Gln Gln Lys Pro Gly Thr Ser Pro Lys Arg Trp Ile Tyr
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys His Gln Arg Ser Ser Tyr Thr Phe Gly
                   85                  90                  95
   Gly Gly Thr Lys Leu Glu Ile Lys
               100
<210> 10
   <211> 117
   <212> PRT
   <213> Artificial Sequence
<220>
   <221> source
   <223> /note="Description of Artificial Sequence: Synthetic polypeptide"
<400> 10
   Glu Val Gln Leu Gln Gln Ser Gly Thr Val Leu Ala Arg Pro Gly Ala
   1               5                   10                  15
   Ser Val Lys Met Ser Cys Lys Ala Ser Gly Tyr Ser Phe Thr Arg Tyr
               20                  25                  30
   Trp Met His Trp Ile Lys Gln Arg Pro Gly Gln Gly Leu Glu Trp Ile
           35                  40                  45
   Gly Ala Ile Tyr Pro Gly Asn Ser Asp Thr Ser Tyr Asn Gln Lys Phe
       50                  55                  60
   Glu Gly Lys Ala Lys Leu Thr Ala Val Thr Ser Ala Ser Thr Ala Tyr
   65                  70                  75                  80
   Met Glu Leu Ser Ser Leu Thr His Glu Asp Ser Ala Val Tyr Tyr Cys
                   85                  90                  95
   Ser Arg Asp Tyr Gly Tyr Tyr Phe Asp Phe Trp Gly Gln Gly Thr Thr
               100                 105                 110
   Leu Thr Val Ser Ser
   115
<210> 11
   <211> 107
   <212> PRT
   <213> Artificial Sequence
<220>
   <221> source
   <223> /note="Description of Artificial Sequence: Synthetic polypeptide"
<400> 11
<210> 12
   <211> 121
   <212> PRT
   <213> Artificial Sequence
<220>
   <221> source
   <223> /note="Description of Artificial Sequence: Synthetic polypeptide"
<400> 12
<210> 13
   <211> 108
   <212> PRT
   <213> Artificial Sequence
<220>
   <221> source
   <223> /note="Description of Artificial Sequence: Synthetic polypeptide"
<400> 13
   Ser Ile Val Met Thr Gln Thr Pro Lys Phe Leu Leu Val Ser Ala Gly
   1 5 10 15
   Asp Arg Val Thr Ile Thr Cys Thr Ala Ser Gln Ser Val Ser Asn Asp
   20 25 30
   Val Val Trp Tyr Gln Gln Lys Pro Gly Gln Ser Pro Lys Met Leu Met
   35 40 45
   Tyr Ser Ala Phe Asn Arg Tyr Thr Gly Val Pro Asp Arg Phe Thr Gly
   50 55 60
   Arg Gly Tyr Gly Thr Asp Phe Thr Phe Thr Ile Ser Ser Val Gln Ala
   65 70 75 80
   Glu Asp Leu Ala Val Tyr Phe Cys Gln Gln Asp Tyr Asn Ser Pro Arg
   85 90 95
   Thr Phe Gly Gly Gly Thr Lys Leu Glu Ile Lys Arg
   100 105
<210> 14
   <211> 117
   <212> PRT
   <213> Artificial Sequence
<220>
   <221> source
   <223> /note="Description of Artificial Sequence: Synthetic polypeptide"
<400> 14
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr His Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Gly Glu Pro Thr Tyr Ala Asp Asp Phe
       50                  55                  60
   Lys Glu His Phe Ala Phe Ser Leu Glu Thr Ser Ala Ser Thr Val Phe
   65                  70                  75                  80
   Leu Gln Ile Asn Asn Leu Lys Asn Glu Asp Thr Ala Thr Tyr Phe Cys
                   85                  90                  95
   Ala Arg Glu Arg Gly Asp Ala Met Asp Tyr Trp Gly Gln Gly Thr Ser
               100                 105                 110
   Val Thr Val Ser Ser
           115
<210> 15
   <211> 107
   <212> PRT
   <213> Artificial Sequence
<220>
   <221> source
   <223> /note="Description of Artificial Sequence: Synthetic polypeptide"
<400> 15
<210> 16
   <211> 118
   <212> PRT
   <213> Artificial Sequence
<220>
   <221> source
   <223> /note="Description of Artificial Sequence: Synthetic polypeptide"
<400> 16
   Gln Val Gln Leu Gln Glu Ser Gly Pro Gly Leu Val Arg Pro Ser Gln
   1               5                   10                  15
   Thr Leu Ser Leu Thr Cys Thr Val Ser Gly Tyr Ser Ile Thr Ser Asp
               20                  25                  30
   His Ala Trp Ser Trp Val Arg Gln Pro Pro Gly Arg Gly Leu Glu Trp
           35                  40                  45
   Ile Gly Tyr Ile Ser Tyr Ser Gly Ile Thr Thr Tyr Asn Pro Ser Leu
       50                  55                  60
   Lys Ser Arg Val Thr Met Leu Arg Asp Thr Ser Lys Asn Gln Phe Ser
   65                  70                  75                  80
   Leu Arg Leu Ser Ser Val Thr Ala Ala Asp Thr Ala Val Tyr Cys Ala
                   85                  90                  95
   Arg Ser Leu Ala Arg Thr Thr Ala Met Asp Tyr Trp Gly Gln Gly Ser
               100                 105                 110
   Leu Val Thr Val Ser Ser
           115
<210> 17
   <211> 103
   <212> PRT
   <213> Artificial Sequence
<220>
   <221> source
   <223> /note="Description of Artificial Sequence: Synthetic polypeptide"
<400> 17
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys Tyr Ala Ser Gln Ser Phe Ser Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Ala Tyr Tyr Cys His Gln Ser Ser Ser Leu Pro Phe
                   85                  90                  95
   Thr Phe Gly Pro Gly Thr Lys
               100
<210> 18
   <211> 115
   <212> PRT
   <213> Artificial Sequence
<220>
   <221> source
   <223> /note="Description of Artificial Sequence: Synthetic polypeptide"
<400> 18
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Val Tyr
               20                  25                  30
   Gly Met Asn Trp Val Arg Gln Ala Pro Gly Lys Gly Leu Glu Trp Val
           35                  40                  45
   Ala Ile Ile Trp Tyr Asp Gly Asp Asn Gln Tyr Tyr Ala Asp Ser Val
       50                  55                  60
   Lys Gly Arg Phe Thr Ile Ser Arg Asp Asn Ser Lys Asn Thr Leu Tyr
   65 70 75 80
   Leu Gln Met Asn Gly Leu Arg Ala Glu Asp Thr Ala Val Tyr Tyr Cys
   85 90 95
   Ala Arg Asp Leu Arg Thr Gly Pro Phe Asp Tyr Trp Gly Gln Gly Thr
   100 105 110
   Leu Val Thr
   115
<210> 19
   <211> 123
   <212> PRT
   <213> Artificial Sequence
<220>
   <221> source
   <223> /note="Description of Artificial Sequence: Synthetic polypeptide"
<400> 19
   Ser Ser Glu Leu Thr Gln Asp Pro Ala Val Ser Val Ala Leu Gly Gln
   1 5 10 15
   Thr Val Arg Val Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Gly Asn Arg Pro Ser Gly Ile Pro Asn Arg Phe Ser Gly Ser Ser
   65 70 75 80
   Ser Gly Asn Ala Ser Leu Thr Ile Thr Gly Ala Gln Ala Glu Asp Glu
   85 90 95
   Ala Asp Tyr Tyr Cys Ser Ser Arg Asp Ser Ser Gly Asn His Trp Val
   100 105 110
   Phe Gly Gly Gly Thr Glu Leu Thr Val Leu Gly
   115 120
<210> 20
   <211> 123
   <212> PRT
   <213> Artificial Sequence
<220>
   <221> source
   <223> /note="Description of Artificial Sequence: Synthetic polypeptide"
<400> 20
   Gln Val Gln Leu Gln Gln Ser Gly Ala Glu Val Lys Lys Pro Gly Ser
   1               5                   10                  15
   Ser Val Arg Val Ser Cys Lys Ala Ser Gly Gly Thr Phe Asn Asn Asn
               20                  25                  30
   Ala Ile Asn Trp Val Arg Gln Ala Pro Gly Gln Gly Leu Glu Trp Met
           35                  40                  45
   Gly Gly Ile Ile Pro Met Phe Gly Thr Ala Lys Tyr Ser Glu Asn Phe
       50                  55                  60
   Gln Gly Arg Val Ala Ile Thr Ala Asp Glu Ser Thr Gly Thr Ala Ser
   65                  70                  75                  80
   Met Glu Leu Ser Ser Leu Arg Ser Glu Asp Thr Ala Val Tyr Tyr Cys
                   85                  90                  95
   Ala Arg Ser Arg Asp Leu Leu Leu Phe Pro His His Ala Leu Ser Pro
               100                 105                 110
   Trp Gly Arg Gly Thr Met Val Thr Val Ser Ser
           115                 120
<210> 21
   <211> 107
   <212> PRT
   <213> Homo sapiens
<400> 21
   Gly Gln Pro Arg Glu Pro Gln Val Tyr Thr Leu Pro Pro Ser Arg Asp
   1               5                   10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 22
   <211> 5
   <212> PRT
   <213> Artificial Sequence
<220>
   <221> source
   <223> /note="Description of Artificial Sequence: Synthetic peptide"
<400> 22
   Ala Asp Ala Ala Pro
   1               5
<210> 23
   <211> 5
   <212> PRT
   <213> Artificial Sequence
<220>
   <221> source
   <223> /note="Description of Artificial Sequence: Synthetic peptide"
<400> 23
   Thr Val Ala Ala Pro
   1               5
<210> 24
   <211> 11
   <212> PRT
   <213> Artificial Sequence
<220>
   <221> source
   <223> /note="Description of Artificial Sequence: Synthetic peptide"
<400> 24
   Ala Asp Ala Ala Pro Thr Val Ser Ile Phe Pro
   1               5                   10
<210> 25
   <211> 12
   <212> PRT
   <213> Artificial Sequence
<220>
   <221> source
   <223> /note="Description of Artificial Sequence: Synthetic peptide"
<400> 25
   Thr Val Ala Ala Pro Ser Val Phe Ile Phe Pro Pro
   1               5                   10
<210> 26
   <211> 6
   <212> PRT
   <213> Artificial Sequence
<220>
   <221> source
   <223> /note="Description of Artificial Sequence: Synthetic peptide"
<400> 26
   Gln Pro Lys Ala Ala Pro
   1               5
<210> 27
   <211> 13
   <212> PRT
   <213> Artificial Sequence
<220>
   <221> source
   <223> /note="Description of Artificial Sequence: Synthetic peptide"
<400> 27
   Gln Pro Lys Ala Ala Pro Ser Val Thr Leu Phe Pro Pro
   1               5                   10
<210> 28
   <211> 5
   <212> PRT
   <213> Artificial Sequence
<220>
   <221> source
   <223> /note="Description of Artificial Sequence: Synthetic peptide"
<400> 28
   Gly Gly Ser Gly Gly
   1               5
<210> 29
   <211> 9
   <212> PRT
   <213> Artificial Sequence
<220>
   <221> source
   <223> /note="Description of Artificial Sequence: Synthetic peptide"
<400> 29
   Gly Gly Ser Gly Gly Gly Gly Ser Gly
   1               5
<210> 30
   <211> 13
   <212> PRT
   <213> Artificial Sequence
<220>
   <221> source
   <223> /note="Description of Artificial Sequence: Synthetic peptide"
<400> 30
   Gly Gly Ser Gly Gly Gly Gly Ser Gly Gly Gly Gly Ser
   1               5                   10
<210> 31
   <211> 6
   <212> PRT
   <213> Artificial Sequence
<220>
   <221> source
   <223> /note="Description of Artificial Sequence: Synthetic peptide"
<400> 31
   Ala Lys Thr Thr Ala Pro
   1               5
<210> 32
   <211> 6
   <212> PRT
   <213> Artificial Sequence
<220>
   <221> source
   <223> /note="Description of Artificial Sequence: Synthetic peptide"
<400> 32
   Ala Ser Thr Lys Gly Pro
   1 5
<210> 33
   <211> 13
   <212> PRT
   <213> Artificial Sequence
<220>
   <221> source
   <223> /note="Description of Artificial Sequence: Synthetic peptide"
<400> 33
   Ala Lys Thr Thr Ala Pro Ser Val Tyr Pro Leu Ala Pro
   1 5 10
<210> 34
   <211> 13
   <212> PRT
   <213> Artificial Sequence
<220>
   <221> source
   <223> /note="Description of Artificial Sequence: Synthetic peptide"
<400> 34
   Ala Ser Thr Lys Gly Pro Ser Val Phe Pro Leu Ala Pro
   1 5 10
<210> 35
   <211> 6
   <212> PRT
   <213> Artificial Sequence
<220>
   <221> source
   <223> /note="Description of Artificial Sequence: Synthetic peptide"
<400> 35
   Gly Gly Gly Gly Ser Gly
   1 5
<210> 36
   <211> 10
   <212> PRT
   <213> Artificial Sequence
<220>
   <221> source
   <223> /note="Description of Artificial Sequence: Synthetic peptide"
<400> 36
   Gly Gly Gly Gly Ser Gly Gly Gly Gly Ser
   1               5                   10
<210> 37
   <211> 14
   <212> PRT
   <213> Artificial Sequence
<220>
   <221> source
   <223> /note="Description of Artificial Sequence: Synthetic peptide"
<400> 37
   Gly Gly Gly Gly Ser Gly Gly Gly Gly Ser Gly Gly Gly Gly
   1               5                   10
<210> 38
   <211> 330
   <212> PRT
   <213> Artificial Sequence
<220>
   <221> source
   <223> /note="Description of Artificial Sequence: Synthetic polypeptide"
<400> 38
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330